# EUROPEAN PATENT SPECIFICATION

(11) **EP 1 598 030 B1**
(45) Date of publication and mention of the grant of the patent: **25.06.2008**
(21) Application number: 05015416.0
(22) Date of filing: 08.02.2001
(51) Int. Cl.: A61F 2/02, A61N 1/378

(54) **Controlled urinary incontinence treatment**
Kontrollierte Harninkontinenzbehandlung
Traitement pour le controle de l'incontinence urinaire

(30) Priority: 10.02.2000 US 181465 P; 10.02.2000 US 181466 P
(43) Date of publication of application: 23.11.2005
(62) Divisional of application: 01904732.3
(73) Proprietor: Potencia Medical AG, 6341 Baar (CH)
(72) Inventor: Forsell, Peter, 6313 Menzingen (CH)
(74) Representative: Wihlsson, Joakim Per Magnus

(56) References cited:
- WO-A-99/63907
- FR-A- 2 756 485
- GB-A- 1 174 814
- US-A- 4 551 862
- US-A- 4 711 231
- US-A- 5 762 599

## Description

The present invention relates to a urinary incontinence treatment apparatus, comprising a restriction device implantable in a patient suffering from urinary incontinence for engaging the urethra or urine bladder to form a restricted urine passageway in the urethra or urine bladder, wherein the restriction device is operable to change the restriction of the urine passageway.

The preamble of claim 1 is defined by US-A-4711231, which defines the relevant state of the art.

Urine incontinence is a widespread problem. Many people are helped through training of the muscles in the pelvic floor but too many have severe problems with urine leakage. Many different solutions to this problem have been tried. For example, there is a prior manually operated urine incontinence treatment apparatus having an artificial hydraulic sphincter device engaging the urethra and connected to an elastic reservoir implanted in the scrotum or in the region of the labia majora. A disadvantage of this prior apparatus is that over time hard fibrosis is developed around the reservoir, which may cause malfunction of pumping components. Furthermore, it is a rather complicated task to manually squeeze the elastic implanted reservoir to pump hydraulic fluid to open the sphincter device when the patient needs to urinate. In particular women can get their fingers wet. The created fibrosis will sooner or later become a hard fibrotic layer, which may make it even more difficult to pump the reservoir. Yet a further disadvantage is that the use of hydraulic fluid always entails a risk of fluid leaking from implanted hydraulic components.

A prior hydraulic apparatus designed to compress the urethra is disclosed in U.S. Pat. No. 5520606. Prosthetic sphincters with an inflateable cuff, which surrounds the urethra or encloses it on two sides is disclosed in for example U.S. Pat. Nos. 4571749 and 4222377. U.S. Pat. No. 4969474 discloses a hydraulic method for treating both men and women with urinary incontinence problems in the same way. The apparatus of U.S. Pat. No. 4969474 includes a reservoir containing fluid and an inflatable compression means designed to compress urethra without risking tissue loss or necrosis to occur. An artificial hydraulically operated urethral sphincter employing an external magnet to achieve closure of the urethral cuff is disclosed in U.S. Pat. No. 5562598.

A prior mechanical prosthetic sphincter, disclosed in U.S. Pat. No. 4619245, comprises a manually controllable actuating component for implanting at a convenient location in the patients body.

The object of the present invention is to provide a new convenient urinary incontinence treatment apparatus, the performance of which may be affected by the patient at any time after operation, in particular when various needs arise over the course of a day, so that the patient substantially always is satisfied or comfortable.

This object is achieved by a urinary incontinence treatment apparatus as defined in claim 1.

The advantage is achieved that the restriction device can be non-invasively operated, when the restriction device has to be adjusted. Furthermore, the apparatus of the invention provides a simple and effective control of the energy supplied to implanted components of the apparatus which ensures an extended and reliable functionality of the apparatus, possibly for the rest of the patient's life and at least many years.

The control device may also control the restriction device. The control device may comprise an internal control unit, preferably including a microprocessor, implantable in the patient for controlling the restriction device. The control device may further comprise an external control unit outside the patient's body, wherein the internal control unit is programmable by the external control unit, for example for controlling the restriction device over time. Alternatively, the internal control unit may control the restriction device over time in accordance with an activity schedule program, which may be adapted to the patient's needs.

A great advantage is that the patient is enabled to adjust the restriction of the urine passageway by using the control device whenever he likes during the day.

Conveniently, the external control unit may load the internal control unit with data in accordance with a loading mode only authorized for a doctor. For specialized controls of the restriction device, the external control unit may control the internal control unit in accordance with a doctor mode only authorized for the doctor. For simple controls of the restriction device, the external control unit may control the internal control unit in accordance with a patient mode permitted for the patient. Thus, by using the external control unit in accordance with different modes it is possible to have certains functions of the restriction device controlled by the patient and other more advanced functions controlled by the doctor, which enables a flexible post-operation treatment of the patient.

The control device may be adapted to control the source of energy to release energy, for instance to intermittently release energy in the form of a train of energy pulses, for direct use in connection with the operation of the restriction device. In accordance with a suitable embodiment the control device controls the source of energy to release electric energy, and the apparatus further comprises an implantable capacitor for producing the train of energy pulses from the released energy. In this case the term *"direct"* is used to mean, on one hand, that the released energy is used while it is being released by the control device, on the other hand, that the released energy may be somewhat delayed, in the order of seconds, by for instance an energy stabilizer before being used in connection with the operation of the restriction device. The restriction device may be operable in non-manual, a non-magnetic or non-mechanical manner by use of the released energy.

In accordance with a preferred embodiment of the invention, the apparatus comprises implantable electrical components including at least one, or only one single voltage level guard and a capacitor or accumulator, wherein the charge and discharge of the capacitor or accumulator is controlled by use of the voltage level guard. As a result, there is no need for any implanted current detector and/or charge level detector for the control of the capacitor, which makes the apparatus simple and reliable.

Generally, the apparatus further comprises an operation device implantable in the patient for operating the restriction device, wherein the control device controls the operation device to operate the restriction device. The control device may directly power the operation device with energy released from the source of energy and/or power other implantable energy consuming components of the apparatus. In this case the term "*directly*" is used to mean, on one hand, that the operation device is powered by released energy while the latter is being released by the control device, on the other hand, that the released energy may be somewhat delayed, in the order of seconds, by for instance an energy stabilizer before powering the operation device. The advantage of directly using energy as it is released is that the apparatus can be of a very simple design and the few components involved makes the apparatus reliable.

The control device may release magnetic, electromagnetic, kinetic, sonic or thermal energy, or non-magnetic, non-sonic, non-thermal, non-electromagnetic or non-kinetic energy.

However, preferably the operation device comprises an electrical operation device.

Typically the apparatus of the invention comprises an adjustment device for adjusting the restriction device to change the restriction of the urine passageway. The adjustment device may be adapted to mechanically adjust the restriction device. Alternatively, the adjustment device may be adapted to hydraulically adjust the restriction device by using hydraulic means which is devoid of hydraulic fluid of the kind having a viscosity that substantially increases when exposed to heat or a magnetic field, i.e. the hydraulic fluid would not become more viscous when exposed to heat or influenced by magnetic forces.

The restriction device may be non-inflatable, i.e. with no hydraulic fluid involved for the adjustments of the restriction device. This eliminates problems with fluid leaking from the restriction device.

The operation device may comprise hydraulic means and at least one valve for controlling a fluid flow in the hydraulic means. The control device may suitably comprise a wireless remote control for controlling the valve. The restriction device may comprise hydraulic means and the operation device may comprise a reservoir forming a fluid chamber with a variable volume connected to the hydraulic means. The operation device may distribute fluid from the chamber to the hydraulic means by reduction of the volume of the chamber and withdraw fluid from the hydraulic means to the chamber by expansion of the volume of the chamber.

In accordance with a first main aspect of the invention, the source of energy is external to the patient's body and the control device controls the source of energy to release wireless energy. The external source of energy may be of any conceivable kind, such as a nuclear source of energy or a chemical source of energy.

An energy storage device, preferably an electric accumulator, may be implantable in the patient for storing the wireless energy released from the external source of energy. The electric accumulator may comprise at least one capacitor or at least one rechargeable battery, or a combination of at least one capacitor and at least one rechargeable battery. Alternatively, a battery may be implantable in the patient for supplying electric energy to implanted electric energy consuming components of the apparatus, in addition to the supply of wireless energy. Where the control device comprises an implantable control unit the electronic circuit thereof and the restriction device may be directly powered by transformed wireless energy, or energy from either the implantable energy storage device or battery.

In accordance with a second main aspect of the invention, the wireless energy is directly used for operation of the restriction device, i.e. the restriction device is operated as the wireless energy is released from the external source of energy by the control device. In this case the term "*directly*" is used to mean, on one hand, that the restriction device is promptly operated by using the released energy whithout first storing the latter, on the other hand, that the released energy may be somewhat delayed, in the order of seconds, by for instance an energy stabilizer before being used for the operation of the restriction device. As a result, a very simple control of the restriction device is achieved and there are only a few implanted components of the apparatus. For example, there is no implanted source of energy, such as a battery, nor any implanted complicated signal control system. This gives the advantage that the apparatus will be extremely reliable.

Generally, the control device controls and directly or indirectly powers the operation device with wireless energy released from the source of energy and/or powers other implanted energy consuming components of the apparatus.

In a first particular embodiment in accordance with the first and second main aspects of the invention, the operation device comprises a motor, preferably an electric motor which may have electrically conductive parts made of plastics. The motor may include a rotary motor, wherein the control device is adapted to control the rotary motor to rotate a desired number of revolutions. Alternatively, the motor may include a linear motor, or a hydraulic or pneumatic fluid motor, wherein the control device is adapted to control the fluid flow through the fluid motor. Motors currently available on the market are getting smaller and smaller. Furthermore, there is a great variety of control methods and miniaturized control equipment available. For example, a number of revolutions of a rotary motor may be analyzed by a Hall-element just a few mm in size.

In a second particular embodiment in accordance with the first and second main aspects of the invention, the control device is adapted to shift polarity of the released energy to reverse the operation device. The operation device may suitably comprise an electric motor and the released energy may comprise electric energy.

In a third particular embodiment in accordance with the first and second main aspects of the invention, the restriction device is operable to perform a reversible function and there is a reversing device implantable in the patient for reversing the function performed by the restriction device. Such a reversing function preferably involves enlarging and restricting the urine passageway by the restriction device, suitably in a stepless manner. In this connection, the control device suitably controls the reversing device, which may include a switch, to reverse the function performed by the restriction device. The reversing device may comprise hydraulic means including a valve for shifting the flow direction of a fluid in the hydraulic means. Alternatively, the reversing device may comprise a mechanical reversing device, such as a switch or a gearbox.

Where the reversing device comprises a switch the control device suitably controls the operation of the switch by shifting polarity of released energy supplied to the switch. The switch may comprise an electric switch and the source of energy may supply electric energy for the operation of the switch. The switch mentioned above may comprise an electronic switch or, where applicable, a mechanical switch.

In accordance with the third particular embodiment, the operation device preferably comprises a motor, wherein the reversing device reverses the motor.

In a fourth particular embodiment in accordance with the first and second main aspects of the invention, the restriction device comprises hydraulic means, for example including an expansible/contractible cavity for fluid. Preferably, the operation device is adapted to conduct hydraulic fluid in the hydraulic means, and comprises a motor, a valveless fluid conduit connected to the hydraulic means of the restriction device, and a reservoir for fluid, wherein the reservoir forms part of the conduit. The operation device suitably comprises a pump operated by the motor. All of the hydraulic components involved are preferably deviod of any non-return valve. This is of great advantage, because with valves involved there is always a risk of malfunction due to inproperly working valves, especially when long time periods passes between valve operations. The reservoir may form a fluid chamber with a variable volume, and the pump may distribute fluid from the chamber to the hydraulic means of the restriction device by reduction of the volume of the chamber and withdraw fluid from the hydraulic means to the chamber by expansion of the volume of the chamber.

In accordance with a third main aspect of the invention, the source of energy is implantable in the patient. Thus, when the source of energy is implanted in a patient the control device controls it from outside the patient's body to release energy. This solution is advantageous for embodiments of the apparatus that have a relatively high consumption of energy, which cannot be satisfied by direct supply of wireless energy.

The implantable source of energy may comprise an accumulator, preferably an electric source of energy, such as a battery having a lifetime of at least 10 years.

In accordance with a fourth main aspect of the invention, the apparatus comprises a switch implanted in the patient for directly or indirectly switching the operation of the restriction device and an internal source of energy, such as a battery, implanted in the patient for supplying energy for the operation of the restriction device, wherein the switch directly or indirectly affects the supply of energy from the internal source of energy. This solution is advantageous for embodiments of the apparatus that have a relatively high energy consumption which cannot be met by direct supply of wireless energy.

In a first particular embodiment in accordance with the fourth main aspect of the invention, the switch switches between an off mode, in which the internal source of energy is not in use, and an on mode, in which the internal source of energy supplies energy for the operation of the restriction device. In this case, the switch is conveniently operated by the wireless energy released from the external source of energy to switch between the on and off modes. The control device, preferably comprising a wireless remote control, may control the external source of energy to release the wireless energy. The advantage of this embodiment is that the lifetime of the implanted source of energy, such as a battery, can be significantly prolonged, since the implanted source of energy does not supply energy when the switch is in its off mode.

In a second particular embodiment in accordance with the fourth main aspect of the invention, the control device comprises a wireless remote control for controlling the internal source of energy. In this case, the switch is operable by the wireless energy from the external source of energy to switch between an off mode, in which the internal source of energy and remote control are not in use, and a standby mode, in which the remote control is permitted to control the internal source of energy to supply energy for the operation of the restriction device.

In a third particular embodiment in accordance with the fourth main aspect of the invention, the apparatus further comprises an energy transforming device implanted in the patient for transforming the wireless energy into storable energy, wherein the internal source of energy is capable of storing the storable energy. The internal source of energy preferably comprises an electric accumulator, at least one capacitor or at least one rechargeable battery, or a combination of at least one capacitor and at least one rechargeable battery. In this case, the switch switches from an off mode, in which the internal source of energy is not in use, to an on mode, in which the internal source of energy supplies energy for the operation of the restriction device.

The control device, preferably comprising a wireless remote control, may control the switch to switch between the on and off modes.

Alternatively, in this third particular embodiment an energy storage device may be implanted in the patient for storing the storable energy instead of the internal source of energy, wherein the switch is operable by energy from the implanted energy storage device to switch between an off mode, in which the internal source of energy is not in use, and an on mode, in which the internal source of energy supplies energy for the operation of the restriction device. In this case, the control device (the wireless remote control) controls the energy storage device to operate the switch.

The internal source of energy preferably comprises an electric source of energy, such as an accumulator or a battery having a lifetime of at least 10 years. However, other kinds of sources are also conceivable, such as a nuclear source of energy or a chemical source of energy.

The above first, second, third and fourth particular embodiments described in connection with the first and second main aspects of the invention are also applicable in accordance with the third main aspect of the invention, i.e. where the source of energy is implantable, and in accordance with the fourth main aspect of the invention, i.e. where the apparatus comprises an implantable switch.

All of the above embodiments may be combined with at least one implantable sensor for sensing at least one physical parameter of the patient, wherein the control device may control the restriction device in response to signals from the sensor. For example, the sensor may comprise a pressure sensor for directly or indirectly sensing the pressure in the urethra or urine bladder. The expression "*indirectly sensing the pressure in the urethra or urine bladder*" should be understood to encompass the cases where the sensor senses the pressure against the restriction device or human tissue of the patient. Where the control device comprises an internal control unit to be implanted in the patient, the internal control unit may suitably directly control the restriction device in response to signals from the sensor. In response to signals from the sensor, for example pressure, the patient's position or any other important physical parameter, the internal control unit may send information thereon to outside the patient's body. The control unit may also automatically control the restriction device in response to signals from the sensor. For example, the control unit may control the restriction device to firmly close the urine passageway in response to the sensor sensing that the patient is lying, or enlarge the urine passageway in response to the sensor sensing an abnormally high pressure against the restriction device.

Where the control device comprises an external control unit outside the patient's body, the external control unit may, suitably directly, control the restriction device in response to signals from the sensor. The external control unit may store information on the physical parameter sensed by the sensor and may be manually operated to control the restriction device based on the stored information. In addition, there may be at least one implantable sender for sending information on the physical parameter sensed by the sensor.

An external data communicator is provided outside the patient's body and an internal data communicator to be implanted in the patient is provided for communicating with the external data communicator. The internal data communicator feeds data related to the patient, or related to the restriction device, back to the external data communicator. Alternatively or in combination, the external data communicator may feed data to the internal data communicator. The internal data communicator may suitably feed data related to at least one physical signal of the patient.

Generally, the apparatus of the invention may comprise a switch implantable in the patient for directly or indirectly switching the energy released from the source of energy. For example, the restriction device may be operable to open and close the urine passageway or may steplessly control the restriction of the urine passageway. A pressure sensor may be provided for directly or indirectly sensing the pressure in the urethra or urine bladder. The control device may control the restriction device in response to signals from the pressure sensor.

The apparatus may comprise an implantable energy transforming device, wherein the control device releases electric energy and the energy transforming device transforms the electric energy into kinetic energy for, preferably direct, operation of the restriction device. Suitably, an implantable stabilizer, such as a capacitor or a rechargeable accumulator, or the like, may be provided for stabilizing the electric energy released by the control device. In addition, the control device may control the source of energy to release energy for a determined time period or in a determined number of energy pulses. Finally, the restriction device may be non-inflatable.

All of the above embodiments are preferably remote controlled. Thus, the control device advantageously comprises a wireless remote control transmitting at least one wireless control signal for controlling the restriction device. With such a remote control it will be possible to adapt the function of the apparatus to the patient's need in a daily basis, which is beneficial with respect to the treatment of the patient.

The wireless remote control may be capable of obtaining information on the condition of the restriction device and of controlling the restriction device in response to the information. Also, The remote control may be capable of sending information related to the restriction device from inside the patient's body to the outside thereof.

In a particular embodiment of the invention, the wireless remote control comprises at least one external signal transmitter or transceiver and at least one internal signal receiver or transceiver implantable in the patient. In another particular embodiment of the invention, the wireless remote control comprises at least one external signal reciever or transceiver and at least one internal signal transmitter or transceiver implantable in the patient.

The remote control may transmit a carrier signal for carrying the control signal, wherein the carrier signal is frequency, amplitude or frequency and amplitude modulated and is digital, analog or digital and analog. Also the control signal used with the carrier signal may be frequency, amplitude or frequency and amplitude modulated.

The control signal may comprise a wave signal, for example, a sound wave signal, such as an ultrasound wave signal, an electromagnetic wave signal, such as an infrared light signal, a visible light signal, an ultra violet light signal, a laser signal, a micro wave signal, a radio wave signal, an x-ray radiation signal, or a gamma radiation signal. Where applicable, two or more of the above signals may be combined.

The control signal may be digital or analog, and may comprise an electric or magnetic field. Suitably, the wireless remote control may transmit an electromagnetic carrier wave signal for carrying the digital or analog control signal. For example, use of an analog carrier wave signal carrying a digital control signal would give safe communication. The control signal may be transmitted in pulses by the wireless remote control.

In all of the above solutions, the control device advantageously releases energy from the source of energy in a non-invasive, magnetic, non-magnetic, mechanical or non-mechanical manner.

The control device may release magnetic, electromagnetic, kinetic or thermal energy, or non-magnetic, non-thermal, non-electromagnetic or non-kinetic energy.

The control device may be activated in a manual or non-manual manner to control the source of energy to release energy.

The above-presented embodiments of the invention may be modified in accordance with the following suggestions. The released energy may comprise electric energy and an implantable capacitor having a capacity less than 0,1 µF may be provided for producing the above-mentioned train of energy pulses.

An implantable motor or pump may be provided for operating the restriction device, wherein the control device is adapted to control the source of energy to directly power the motor or pump with the released energy. Specifically, the control device may be adapted to release wireless energy in the form of a magnetic field or electromagnetic waves (excluding radio waves) for direct power of the motor or pump, as the wireless energy is being released. Where a pump is used it preferably is not a plunger type of pump.

Generally, the wireless energy comprises a signal.

The apparatus may further comprise implantable energy transforming device for transforming wireless energy directly or indirectly into energy different than the wireless energy, for operation of the restriction device. For example, the motor or pump may be powered by the transformed energy.

The energy transforming device may transform the wireless energy in the form of sound waves, preferably directly, into electric energy for operation of the restriction device. The energy transforming device may comprise a capacitor adapted to produce electric pulses from the transformed electric energy.

The motor mentioned in the present specification may also be directly powered with wirelessly transmitted electromagnetic or magnetic energy in the form of signals, as the energy is transmitted. Furthermore, all the various functions of the motor and associated components described in the present specification may be used where applicable.

Generally, the restriction device advantageously is embedded in a soft or gel-like material, such as a silicone material having hardness less than 20 Shore.

Of course, the restriction device preferably is adjustable in a non-manual manner.

All the above described various components, such as the motor, pump and capacitor, may be combined in the different embodiments where applicable. Also the various functions described in connection with the above embodiments of the invention may be used in different applications, where applicable.

All the various ways of transferring energy and controlling the energy presented in the present specification may be practised by using all of the various components and solutions described.

The present invention may be used in methods for treating urinary incontinent patients.

In a first alternative method, there is a method of treating a patient suffering from urinary incontinence, comprising the steps of implanting an operable restriction device in the patient, so that the restriction device engages the urethra or urine bladder to form a restricted urine passageway in the urethra or urine bladder, providing a source of energy for energizing the restriction device, and controlling the source of energy to release energy for use in connection with the operation of the restriction device. The method may further comprise using energy released from the source of energy to operate the restriction device to open and close, respectively, the urine passageway.

In a second alternative method, there is a method of treating a patient suffering from urinary incontinence, comprising the steps of placing at least two laparascopical trocars in the patient's body, inserting a dissecting tool through the trocars and dissecting an area of the urethra or urine bladder, placing an operable restriction device in the dissected area, so that the restriction device engages the urethra or urine bladder to form a restricted urine passageway in the urethra or urine bladder, implanting a source of energy in the patient, and controlling the implanted source of energy from outside the patient's body to release energy for use in connection with the operation of the restriction device.

In a third alternative method, there is a method of treating a patient suffering from urinary incontinence comprising: (a) Surgically implanting in the patient an operable restriction device engaging the patient's urethra or urine bladder to form a restricted urine passageway in the urethra or urine bladder. (b) Providing a source of energy external to the patient's body. (c) Controlling the external source of energy from outside the patient's body to release wireless energy. And (d) using the released wireless energy in connection with the operation of the restriction device.

The method may further comprise (e) implanting in the human or animal an operation device which can adjust the restricted urine passageway in response to supplied energy, and (f) using the released wireless energy to activate the implanted operation device so as (i) to enlarge the restricted urine passageway to allow urine to readily pass therethrough but normally restrict the urine passageway. In the method (f) may be practiced at least once a day, normally several times (e.g. 2-10) a day.

In a fourth alternative method, there is a method of treating a patient suffering from urinary incontinence, comprising the steps of placing at least two laparascopical trocars in the patient's body, inserting a dissecting tool through the trocars and dissecting an area of the urethra or urine bladder, placing an operable restriction device in the dissected area, so that the restriction device engages the urethra or urine bladder to form a restricted urine passageway in the urethra or urine bladder, providing an external source of energy outside the patient's body, controlling the external source of energy from outside the patient's body to release wireless energy, and using the released wireless energy in connection with the operation of the restriction device.

In a fifth alternative method, there is a method of treating a patient suffering from urinary incontinence, comprising the steps of placing at least two laparascopical trocars in the patient's body, inserting a dissecting tool through the trocars and dissecting an area of the urethra or urine bladder, implanting an operable restriction device in the dissected area, so that the restriction device engages the urethra or urine bladder to form a restricted urine passageway in the urethra or urine bladder, implanting an energy transforming device, providing an external source of energy, controlling the external source of energy to release wireless energy, and transforming the wireless energy by the energy transforming device into energy different than the wireless energy for use in connection with the operation of the restriction device. This method may further comprise implanting a stabilizer in the patient for stabilizing the energy transformed by the energy transforming device.

The invention is described in more detail in the following with reference to the accompanying drawings, in which
FIGURES 1 to 6 are schematic block diagrams illustrating six embodiments, respectively, of the invention, in which wireless energy released from an external source of energy is used for direct operation of a restriction device engaging the urethra or urine bladder of a patient;
FIGURES 7 to 10 are schematic block diagrams illustrating four embodiments, respectively, of the invention, in which energy is released from an implanted source of energy;
FIGURES 11 to 15 are schematic block diagrams illustrating five embodiments, respectively, of the invention, in which a switch is implanted in the patient for directly or indirectly switching the operation of the restriction device;
FIGURE 16 is a schematic block diagram illustrating conceivable combinations of implantable components for achieving various communication options;
FIGURE 17 illustrates the apparatus in accordance with the invention implanted in a patient;
FIGURE 18 is a block diagram illustrating remote control components of an embodiment of the invention; and
FIGURE 19 is a schematic view of exemplary circuitry used for the components of the block diagram of FIGURE 18.

Referring to the drawing figures, like reference numerals designate identical or corresponding elements throughout the several figures.

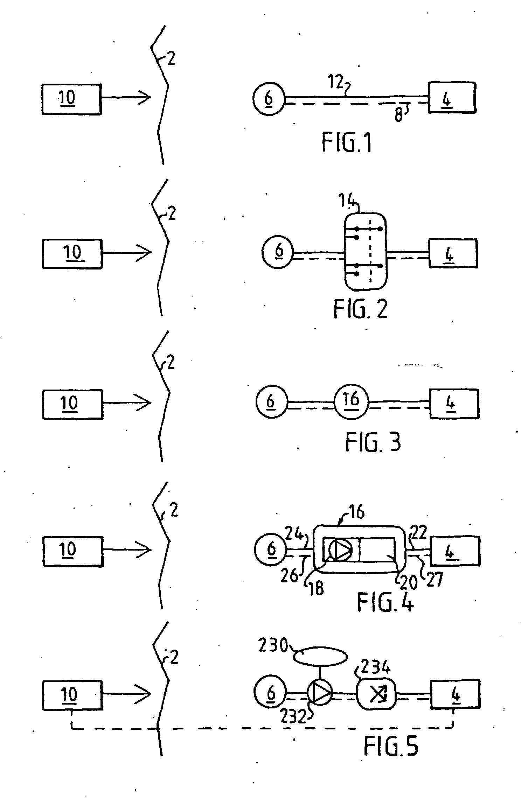

FIGURE 1 schematically shows an embodiment of the urinary incontinence treatment apparatus of the invention having some parts implanted in a patient and other parts located outside the patient's body. Thus, in FIGURE 1 all parts placed to the right of the patient's skin 2 are implanted and all parts placed to the left of the skin 2 are located outside the patient's body. The apparatus of FIGURE 1 comprises an implanted operable restriction device 4, which engages the patient's urethra (or alternatively the urine bladder) to form a restricted urine passageway. The restriction device 4 is capable of performing a reversible function, i.e. to open and close the urine passageway. An implanted control unit 6 controls the restriction device 4 via a control line 8 to form an adequate restriction of the of the urine passageway. An external control unit 10 includes an external source of energy and a wireless remote control transmitting a control signal generated by the external source of energy. The control signal is received by a signal receiver incorporated in the implanted control unit 6, whereby the control unit 6 controls the implanted restriction device 4 in response to the control signal. The implanted control unit 6 also uses energy from the control signal for operating the restriction device 4 via a power supply line 12.

FIGURE 2 shows an embodiment of the invention identical to that of FIGURE 1, except that a reversing device in the form of a switch 14 operable by energy also is implanted in the patient for reversing the restriction device 4. The control unit 6 uses the switch 14 to reverse the function performed by the restriction device 4. More precisely, the external control unit 10 releases energy carried by a wireless signal and the implanted control unit 6 transforms the wireless energy into a current for operating the switch 14. When the control unit 6 shifts the polarity of the current the switch 14 reverses the function performed by the restriction device 4.

FIGURE 3 shows an embodiment of the invention identical to that of FIGURE 1, except that an operation device in the form of a motor 16 also is implanted in the patient. The implanted control unit 6 powers the motor 16 with wireless energy released from the external source of energy of the external control unit 10. The implanted control unit 6 controls the operation of the motor 16 in response to a control signal from the remote control of the external control unit 10.

FIGURE 4 shows an embodiment of the invention identical to that of FIGURE 1, except that an assembly 16 including a motor/pump unit 18 and a fluid reservoir 20 also is implanted in the patient. In this case the restriction device 4 is hydraulically operated, i.e. hydraulic fluid is pumped by the motor/pump unit 18 from the reservoir 20 through a conduit 22 to the restriction device 4 to restrict the urine passageway, and hydraulic fluid is pumped by the motor/pump unit 18 back from the restriction device 4 to the reservoir 20 to enlarge the urine passageway. The external control unit 10 releases energy carried by a wireless signal and the implanted control unit 6 transforms the wireless energy into a current, for example a current, for powering the motor/pump unit 18 via an electric power supply line 24. The implanted control unit 6 controls the motor/pump unit 16 and the restriction device 4 via control lines 26 and 27.

FIGURE 5 shows an embodiment of the invention comprising the restriction device 4, hydraulically operated, and the implanted control unit 6, and further comprising a hydraulic fluid reservoir 230, a motor/pump unit 232 and a reversing device in the form of a hydraulic valve shifting device 234, all of which are implanted in the patient. The motor of the motor/pump unit 232 is an electric motor.

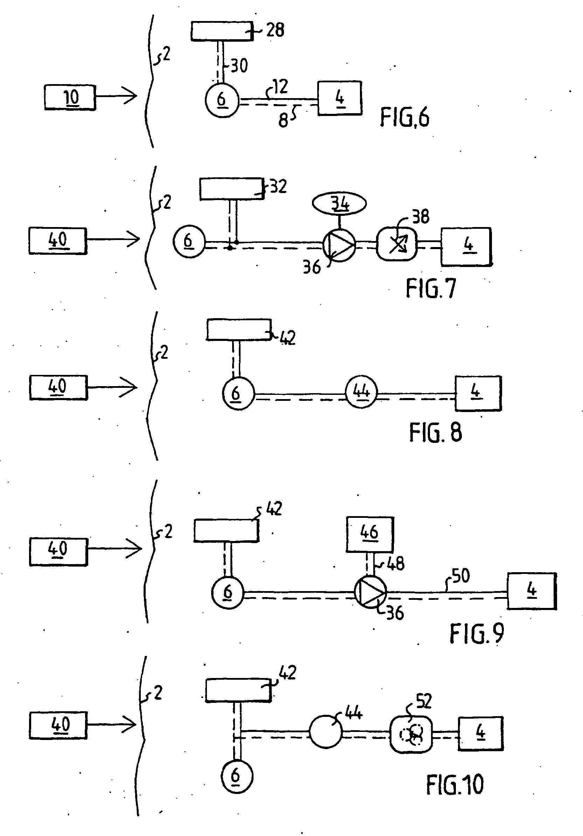

FIGURE 6 shows an embodiment of the invention identical to that of FIGURE 1, except that an accumulator 28 also is implanted in the patient. The control unit 6 stores energy received from the external control unit 10 in the accumulator 28. In response to a control signal from the external control unit 10 the implanted control unit 6 releases energy from the accumulator 28 via a power line 30 for the operation of the restriction device 4.

FIGURE 7 shows an embodiment of the invention comprising the restriction device 4, hydraulically operated, and the implanted control unit 6, and further comprising a source of energy in the form of a battery 32, a hydraulic fluid reservoir 34, a motor/pump unit 36 and a reversing device in the form of a hydraulic valve shifting device 38, all of which are implanted in the patient. The motor of the motor/pump unit 36 is an electric motor. An external control unit 40 includes a wireless remote control transmitting a control signal which is received by the signal receiver incorporated in the implanted control unit 6.

In response to a control signal from the external control unit 40 the implanted control unit 6 powers the motor/pump unit 36 with energy from the battery 32, whereby the motor/pump unit 36 distributes hydraulic fluid between the reservoir 34 and the restriction device 4. The control unit 6 controls the shifting device 38 to shift the hydraulic fluid flow direction between one direction in which the fluid is pumped by the motor/pump unit 36 from the reservoir 34 to the restriction device 4 to restrict the urine passageway, and another opposite direction in which the fluid is pumped by the motor/pump unit 36 back from the restriction device 4 to the reservoir 34 to enlarge the urine passageway.

FIGURE 8 shows an embodiment of the invention identical to that of FIGURE 6, except that a battery 42 is substituted for the accumulator 28, the external control unit 40 of the embodiment of FIGURE 5 is substituted for the external control unit 10 and an electric motor 44 is implanted in the patient for operating the restriction device 4. In response to a control signal from the external control unit 40 the implanted control unit 6 powers the motor 44 with energy from the battery 42, whereby the motor 44 operates the restriction device 4.

FIGURE 9 shows an embodiment of the invention identical to that of FIGURE 8, except that the motor/pump unit 36 of the embodiment of FIGURE 7 is substituted for the motor 44 and a fluid reservoir 46 also is implanted in the patient. The reservoir 46 is via fluid conduits 48 and 50 connected to the motor/pump unit 36 and restriction device 4, which in this case is hydraulically operated. In response to a control signal from the external control unit 40, the implanted control unit 6 powers the electric motor of the motor/pump unit 36 with energy from the battery 42, whereby the motor/pump unit 36 distributes hydraulic fluid between the fluid reservoir 46 and the restriction device 4.

FIGURE 10 shows an embodiment of the invention identical to that of FIGURE 8, except that a mechanical reversing device in the form of a gearbox 52 also is implanted in the patient. The implanted control unit 6 controls the gearbox 52 to reverse the function performed by the restriction device 4 (mechanically operated).

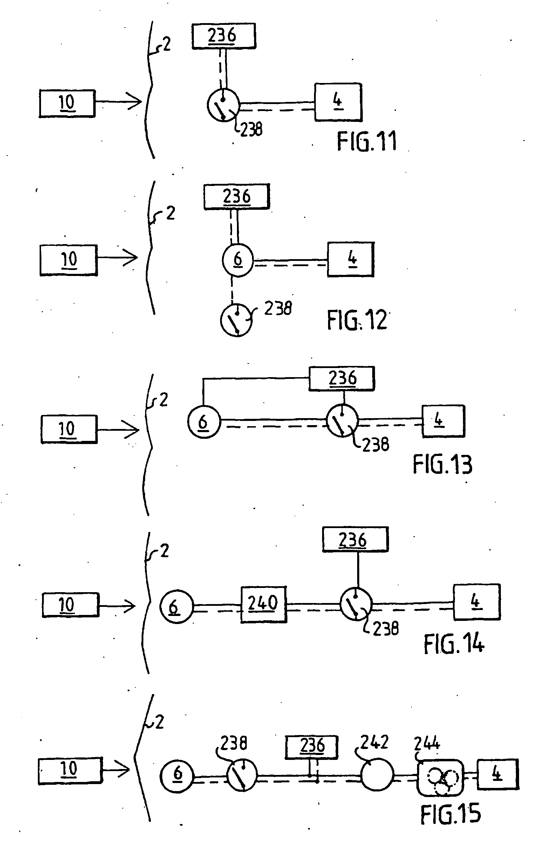

FIGURE 11 shows an embodiment of the invention comprising the restriction device 4, the external control unit 10, an implanted source of energy 236 and an implanted switch 238. The switch 238 is operated by wireless energy released from the external source of energy of the external control unit 6 to switch between an off mode, in which the implanted source of energy 236 is not in use, and an on mode, in which the implanted source of energy 236 supplies energy for the operation of the restriction device 4.

FIGURE 12 shows an embodiment of the invention identical to that of FIGURE 11, except that also the control unit 6 is implanted, in order to receive a control signal from the wireless remote control of the external control unit 10. The switch 238 is operated by the wireless energy from the external source of energy 10 to switch between an off mode, in which the implanted source of energy 236 and the wireless remote control of the external control unit 10 are not in use, i.e. the control unit 6 is not capable of receiving the control signal, and a standby mode, in which the wireless remote control is permitted to control the internal source of energy 236, via the implanted control unit 6, to supply energy for the operation of the restriction device 4.

FIGURE 13 shows an embodiment of the invention identical to that of FIGURE 12, except that an energy transforming device for transforming the wireless energy into storable energy is incorporated in the implanted control unit 6 and that the implanted source of energy 236 is of a type that is capable of storing the storable energy. In this case, in response to a control signal from the external control unit 10, the implanted control unit 6 controls the switch 238 to switch from an off mode, in which the implanted source of energy 236 is not in use, to an on mode, in which the source of energy 36 supplies energy for the operation of the restriction device 4.

FIGURE 14 shows an embodiment of the invention identical to that of FIGURE 13, except that an energy storage device 240 also is implanted in the patient for storing the storable energy transformed from the wireless energy by the transforming device of the control unit 6. In this case, the implanted ontrol unit 6 controls the energy storage device 240 to operate the switch 238 to switch between an off mode, in which the implanted source of energy 236 is not in use, and an on mode, in which the implanted source of energy 236 supplies energy for the operation of the restriction device 4.

FIGURE 15 shows an embodiment of the invention identical to that of FIGURE 13, except that a motor 242 and a mechanical reversing device in the form of a gearbox 244 also are implanted in the patient. The implanted control unit 6 controls the gearbox 244 to reverse the function performed by the restriction device 4 (mechanically operated), i.e. enlarging and restricting the urine passageway.

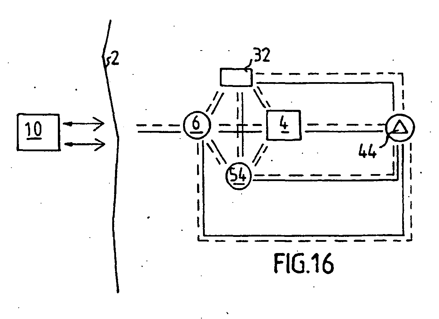

FIGURE 16 schematically shows conceivable combinations of implanted components of the apparatus for achieving various communication possibilities. Basically, there are the implanted restriction device 4, the implanted control unit 6 and the external control unit 10 including the external source of energy and the wireless remote control. As already described above the remote control transmits a control signal generated by the external source of energy, and the control signal is received by a signal receiver incorporated in the implanted control unit 6, whereby the control unit 6 controls the implanted restriction device 4 in response to the control signal.

A sensor 54 may be implanted in the patient for sensing a physical parameter of the patient, such as the pressure in the stomach. The control unit 6, or alternatively the external control unit 10, may control the restriction device 4 in response to signals from the sensor 54. A transceiver may be combined with the sensor 54 for sending information on the sensed physical parameter to the external control unit 10. The wireless remote control of the external control unit 10 may comprise a signal transmitter or transceiver and the implanted control unit 6 may comprise a signal receiver or transceiver. Alternatively, the wireless remote control of the external control unit 10 may comprise a signal receiver or transceiver and the implanted control unit 6 may comprise a signal transmitter or transceiver. The above transceivers, transmitters and receivers may be used for sending information or data related to the restriction device from inside the patient's body to the outside thereof.

The motor 44 may be implanted for operating the restriction device 4 and also the battery 32 may be implanted for powering the motor 44. The battery 32 may be equipped with a transceiver for sending information on the charge condition of the battery.

Those skilled in the art will realize that the above various embodiments according to FIGURES 1-15 could be combined in many different ways. For example, the energy operated switch 14 could be incorporated in any of the embodiments of FIGURES 4,6,8-10. The hydraulic shifting device 38 could be incorporated in any of the embodiments of FIGURES 4 and 9. The gearbox 52 could be incorporated in any of the embodiments of FIGURES 1,6 and 8.

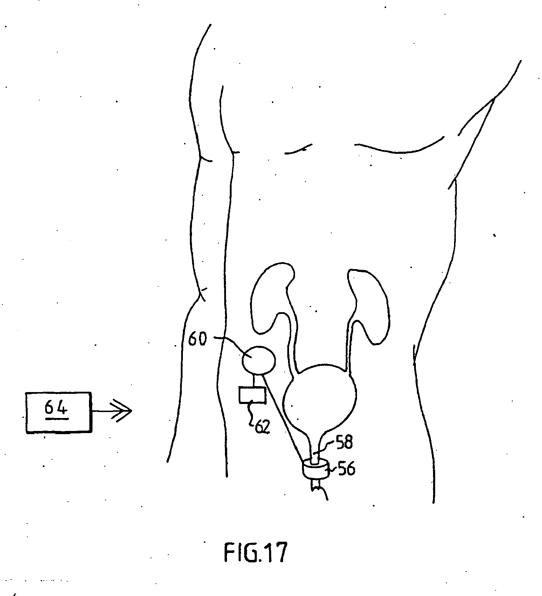

FIGURE 17 illustrates how any of the above-described embodiments of the apparatus of the invention may be implanted in a patient. Thus, an assembly of the apparatus implanted in the patient comprises a restriction device 56 engaging the urethra 58, an operation device 60 for operating the restriction device 56 and an internal control unit 62, which includes a signal receiver, for controlling the operation device 61. An external control unit 64 includes a signal transmitter for transmitting a wireless control signal to the signal receiver of the implanted control unit 62. The implanted control unit 62 is capable of transforming signal energy from the control signal into electric energy for powering the operation device 60 and for energizing energy consuming implanted components of the apparatus.

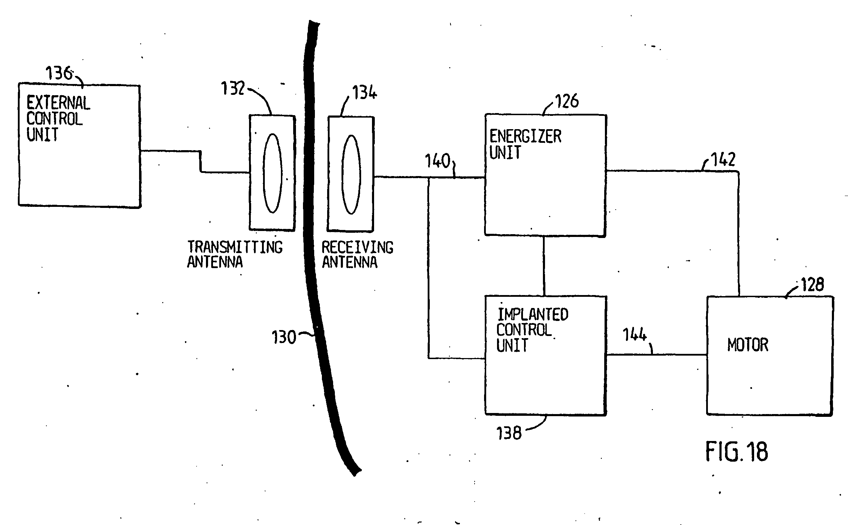

FIGURE 18 shows the basic parts of a wireless remote control of the apparatus of the invention including an electric motor 128 for operating a restriction device, for example of the type illustrated in FIGURE 17. In this case, the remote control is based on the transmission of electromagnetic wave signals, often of high frequencies in the order of 100 kHz - 1 gHz, through the skin 130 of the patient. In FIGURE 18, all parts placed to the left of the skin 130 are located outside the patient's body and all parts placed to the right of the skin 130 are implanted. Any suitable remote control system may be used.

An external signal transmitting antenna 132 is to be positioned close to a signal receiving antenna 134 implanted close to the skin 130. As an alternative, the receiving antenna 134 may be placed for example inside the abdomen of the patient. The receiving antenna 134 comprises a coil, approximately 1-100 mm, preferably 25 mm in diameter, wound with a very thin wire and tuned with a capacitor to a specific high frequency. A small coil is chosen if it is to be implanted under the skin of the patient and a large coil is chosen if it is to be implanted in the abdomen of the patient. The transmitting antenna 132 comprises a coil having about the same restriction as the coil of the receiving antenna 134 but wound with a thick wire that can handle the larger currents that is necessary. The coil of the transmitting antenna 132 is tuned to the same specific high frequency as the coil of the receiving antenna 134.

An external control unit 136 comprises a microprocessor, a high frequency electromagnetic wave signal generator and a power amplifier. The microprocessor of the control unit 136 is adapted to switch the generator on/off and to modulate signals generated by the generator to send digital information via the power amplifier and the antennas 132,134 to an implanted control unit 138. To avoid that accidental random high frequency fields trigger control commands, digital signal codes are used. A conventional keypad placed on the external control unit 136 is connected to the microprocessor thereof. The keypad is used to order the microprocessor to send digital signals to activate the restriction device to either restrict or enlarge the urine passageway. The microprocessor starts a command by applying a high frequency signal on the antenna 132. After a short time, when the signal has energized the implanted parts of the control system, commands are sent to restrict or enlarge the urine passageway in predefined steps. The commands are sent as digital packets in the form illustrated below.

| | | | |
|---|---|---|---|
| Start pattern, 8 bits | Command, 8 bits | Count, 8 bits | Checksum, 8 bits |

The commands are sent continuously during a rather long time period (e.g. about 30 seconds or more). When a new restrict or enlarge step is desired the Count byte is increased by one to allow the implanted control unit 138 to decode and understand that another step is demanded by the external control unit 136. If any part of the digital packet is erroneous, its content is simply ignored.

Through a line 140, an implanted energizer unit 126 draws energy from the high frequency electromagnetic wave signals received by the receiving antenna 134. The energizer unit 126 stores the energy in a power supply, such as a large capacitor, powers the control unit 138 and powers the electric motor 128 via a line 142.

The control unit 138 comprises a demodulator and a microprocessor. The demodulator demodulates digital signals sent from the external control unit 136. The microprocessor of the control unit 138 receives the digital packet, decodes it and, provided that the power supply of the energizer unit 126 has sufficient energy stored, sends a signal via a signal line 144 to the motor 128 to either contract or enlarge the restriction device depending on the received command code.

Alternatively, the energy stored in the power supply of the energizer unit may only be used for powering a switch, and the energy for powering the motor 128 may be obtained from another implanted power source of relatively high capacity, for example a battery. In this case the switch is adapted to connect said battery to the control unit 138 in an on mode when said switch is powered by said power supply and to keep said battery disconnected from the control unit in a standby mode when said switch is unpowered.

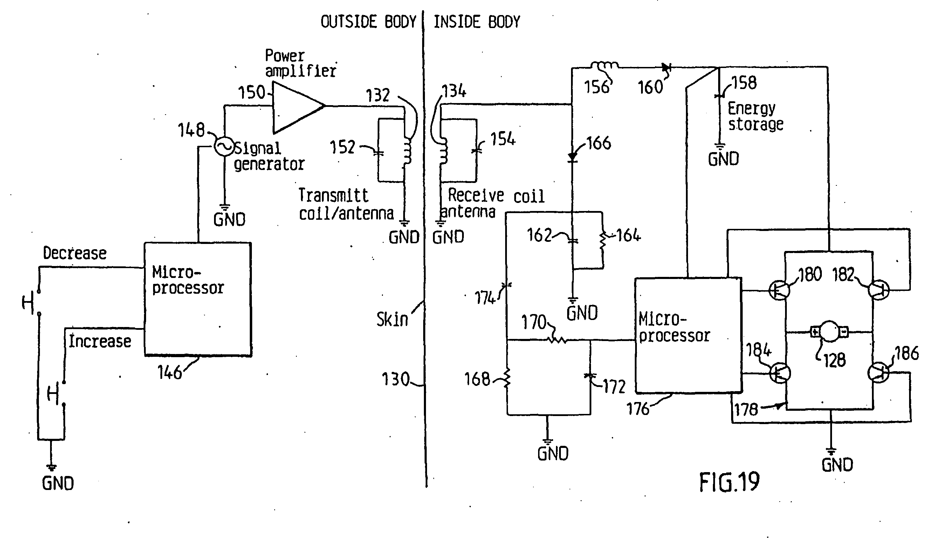

With reference to FIGURE 19, the remote control schematically described above will now be described in accordance with a more detailed embodiment. The external control unit 136 comprises a microprocessor 146, a signal generator 148 and a power amplifier 150 connected thereto. The microprocessor 146 is adapted to switch the signal generator 148 on/off and to modulate signals generated by the signal generator 148 with digital commands that are sent to implanted components of the apparatus. The power amplifier 150 amplifies the signals and sends them to the external signal transmitting antenna 132. The antenna 132 is connected in parallel with a capacitor 152 to form a resonant circuit tuned to the frequency generated by the signal generator 148.

The implanted signal receiving antenna coil 134 forms together with a capacitor 154 a resonant circuit that is tuned to the same frequency as the transmitting antenna 132. The signal receiving antenna coil 134 induces a current from the received high frequency electromagnetic waves and a rectifying diode 160 rectifies the induced current, which charges a storage capacitor 158. A coil 156 connected between the antenna coil 134 and the diode 160 prevents the capacitor 158 and the diode 160 from loading the circuit of the signal receiving antenna 134 at higher frequencies. Thus, the coil 156 makes it possible to charge the capacitor 158 and to transmit digital information using amplitude modulation.

A capacitor 162 and a resistor 164 connected in parallel and a diode 166 forms a detector used to detect amplitude modulated digital information. A filter circuit is formed by a resistor 168 connected in series with a resistor 170 connected in series with a capacitor 172 connected in series with the resistor 168 via ground, and a capacitor 174, one terminal of which is connected between the resistors 168,170 and the other terminal of which is connected between the diode 166 and the circuit formed by the capacitor 162 and resistor 164. The filter circuit is used to filter out undesired low and high frequencies. The detected and filtered signals are fed to an implanted microprocessor 176 that decodes the digital information and controls the motor 128 via an H-bridge 178 comprising transistors 180,182,184 and 186. The motor 128 can be driven in two opposite directions by the H-bridge 178.

The microprocessor 176 also monitors the amount of stored energy in the storage capacitor 158. Before sending signals to activate the motor 128, the microprocessor 176 checks whether the energy stored in the storage capacitor 158 is enough. If the stored energy is not enough to perform the requested operation, the microprocessor 176 waits for the received signals to charge the storage capacitor 158 before activating the motor 128.

## Claims

1. A urine incontinence treatment apparatus, comprising:
a restriction device (4, 56) for engaging the urethra or urine bladder (58) to form a restricted urine passageway in the urethra or urine bladder (58), the restriction device being operable to change the restriction of the urine passageway,
a source of energy (32, 42, 236), and
a control device (6, 10) operable from outside the patient's body for controlling the source of energy to release energy for use in connection with the operation of the restriction device (4, 56), the apparatus comprising:
a motor or pump (16, 18, 36, 232) implantable in the patient, wherein the source of energy (32, 42, 236) is adapted to power the motor or pump (16, 18, 36, 232), and the control device (6, 10) is adapted to control the motor or pump (16, 18, 36, 232) to operate the restriction device (4, 56),
**characterized in that** the apparatus comprises:
an external data communicator intended to be outside the patient's body, and
an internal data communicator implantable in the patient for communicating with the external communicator, wherein the internal data communicator feeds data related to the patient back to the external data communicator.

2. The apparatus according to claim 1, wherein the source of energy is intended to be external to the patient's body when the restriction device (4, 56) is implanted therein, and the control device (6, 10) is adapted to control the external source of energy to release wireless energy for use in connection with the operation of the restriction device (4, 56).

3. The apparatus according to claim 2, wherein the control device (6, 10) is adapted to control the external source of energy to release wireless energy for direct use in connection with the operation of the restriction device (4, 56).

4. The apparatus according to claim 2 or 3, wherein the control device (6, 10) is adapted to control the external source of energy to intermittently release wireless energy in the form of a train of energy pulses for direct use in connection with the operation of the restriction device (4, 56).

5. The apparatus according to any of claims 2-4, wherein the restriction device (4, 56) is operable in a non-magnetic, non-thermal, non-manual or non-mechanical manner by use of said released wireless energy.

6. The apparatus according to claim 1, wherein the restriction device (4, 56) is operable by the released energy in a manual, mechanical, thermal or magnetic manner.

7. The apparatus according to claim 1, wherein the restriction device (4, 56) is operable by the released energy in a non-manual, non-mechanical, non-thermal or non-magnetic manner.

8. The apparatus according to claim 1 or 2, further comprising an operation device (60) implantable in the patient adapted to operate the restriction device (4, 56).

9. The apparatus according to claim 8, wherein the operation device (60) is powered by magnetic energy, non-magnetic energy, electromagnetic energy, non-electromagnetic energy, kinetic energy, non-kinetic energy, thermal energy or non-thermal energy.

10. The apparatus according to claim 8, wherein the operation device (60) comprises an electrical operation device (60).

11. The apparatus according to claim 1 or 2, wherein the control device (6, 10) is activated in a manual or non-manual manner to control the source of energy to release energy.

12. The apparatus according to claim 1, further comprising an adjustment device for adjusting the restriction device (4, 56) to change the restriction of the urine passageway, wherein the adjustment device is adapted to mechanically adjust the restriction device (4, 56), or adapted to hydraulically adjust the restriction device (4, 56) by using hydraulic means which is devoid of hydraulic fluid of the kind having a viscosity that substantially increases when exposed to heat or a magnetic field.

13. The apparatus according to any of claims 1-12, wherein the control device (6, 10) is adapted to control the restriction device (4, 56).

14. The apparatus according to claim 13, wherein the control device (6, 10) comprises an internal control unit (138) implantable in the patient for controlling the restriction device (4, 56).

15. The apparatus according to claim 14, wherein the internal control unit (138) is programmable.

16. The apparatus according to claim 15, wherein the control device (6, 10) comprises an external control unit (136) intended to be outside the patient's body, the internal control unit (138) being programmable by the external control unit (136).

17. The apparatus according to claim 15, wherein the internal control unit (138) is programmable for controlling the restriction device (4, 56) over time.

18. The apparatus according to claim 17, wherein the internal control unit (138) is adapted to control the restriction device (4, 56) over time in accordance with an activity schedule program.

19. The apparatus according to claim 17, wherein the internal control unit (138) comprises a microprocessor (176).

20. The apparatus according to claim 16, wherein the external control unit (136) loads the internal control unit (138) with data in accordance with a loading mode only authorized for a doctor.

21. The apparatus according to claim 16, wherein the external control unit (136) is adapted to control the internal control unit (138) in accordance with a doctor mode only authorized for a doctor.

22. The apparatus according to claim 16, wherein the external control unit (136) is adapted to control the internal control unit (138) in accordance with a patient mode permitted for the patient.

23. The apparatus according to claim 1, wherein the source of energy is implantable in the patient.

24. The apparatus according to claim 23, wherein the implantable source of energy comprises at least one accumulator, at least one capacitor or at least one rechargeable battery, or a combination of at least one capacitor and at least one rechargeable battery.

25. The apparatus according to claim 24, wherein the implantable source of energy comprises an electric source of energy.

26. The apparatus according to claim 25, wherein the electric source of energy comprises an accumulator, or a battery having a lifetime of at least 10 years.

27. The apparatus according to claim 2, further comprising an energy storage device implantable in the patient for storing the wireless energy released from the external source of energy.

28. The apparatus according to claim 27, wherein the energy storage device comprises an accumulator.

29. The apparatus according to claim 28, wherein the accumulator comprises an electric accumulator.

30. The apparatus according to claim 29, wherein the electric accumulator comprises at least one capacitor or at least one rechargeable battery, or a combination of at least one capacitor and at least one rechargeable battery.

31. The apparatus according to claim 2, further comprising a battery implantable in the patient for supplying electric energy to implantable electric energy consuming components of the apparatus.

32. The apparatus according to any one of claims 1, 2, 13 and 23, further comprising a switch (238) implantable in the patient for directly or indirectly switching the operation of the restriction device (4, 56).

33. The apparatus according to claim 32, further comprising an internal source of energy implantable in the patient for supplying energy for the operation of the restriction device (4, 56), wherein the switch (238) directly or indirectly affects the supply of energy from the internal source of energy.

34. The apparatus according to claim 33, wherein the switch (238) is adapted to switch between an "off" mode, in which the internal source of energy is not in use, and an "on" mode, in which the internal source of energy supplies energy for the operation of the restriction device (4, 56).

35. The apparatus according to claim 34, wherein the switch (238) is operable by the wireless energy released from the external source of energy.

36. The apparatus according to any one of claims 1, 2 and 35, wherein the control device (6, 10) comprises a wireless remote control.

37. The apparatus according to claim 33, wherein the control device (6, 10) comprises a wireless remote control for controlling the internal source of energy.

38. The apparatus according to claim 37, wherein the switch (238) is operable by the wireless energy from the external source of energy to switch between an "off" mode, in which the internal source of energy and remote control are not in use, and a "standby" mode, in which the remote control is permitted to control the internal source of energy to supply energy for the operation of the restriction device (4, 56).

39. The apparatus according to claim 33, further comprising an energy transforming device (6, 62) implantable in the patient for transforming the wireless energy into storable energy and an energy storage device implantable in the patient for storing the storable energy.

40. The apparatus according to claim 39, wherein the switch (238) is operable by energy from the implantable energy storage device to switch between an "off" mode, in which the internal source of energy is not in use, and an "on" mode, in which the internal source of energy supplies energy for the operation of the restriction device (4, 56).

41. The apparatus according to claim 40, wherein the control device (6, 10) is adapted to control the energy storage device to operate the switch (238).

42. The apparatus according to claim 13 or 41, wherein the control device (6, 10) comprises a wireless remote control.

43. The apparatus according to claim 33, further comprising an energy transforming device (6, 62) implantable in the patient for transforming the wireless energy into storable energy, wherein the internal source of energy is capable of storing the storable energy.

44. The apparatus according to claim 43, wherein the switch (238) switches from an "off" mode, in which the internal source of energy is not in use, to an "on" mode, in which the source of energy supplies energy for the operation of the restriction device (4, 56).

45. The apparatus according to claim 44, wherein the control device (6, 10) is adapted to control the switch (238) to switch between the "on" and "off" modes.

46. The apparatus according to claim 45, wherein the control device (6, 10) comprises a wireless remote control.

47. The apparatus according to claim 33, wherein the internal source of energy comprises an electric source of energy.

48. The apparatus according to claim 47, wherein the electric source of energy comprises at least one accumulator, at least one capacitor or at least one rechargeable battery, or a combination of at least one capacitor and at least one rechargeable battery.

49. The apparatus according to claim 47, wherein the electric source of energy comprises an accumulator or a battery having a lifetime of at least 10 years.

50. The apparatus according to claim 1, wherein the source of energy comprises a nuclear source of energy.

51. The apparatus according to claim 1, wherein the source of energy comprises a chemical source of energy.

52. The apparatus according to claim 3, further comprising an operation device (60) implantable in the patient for operating the restriction device (4, 56), wherein the wireless energy directly or indirectly powers the operation device (60).

53. The apparatus according to claim 8 or 52, wherein the control device (6, 10) is adapted to control the operation device (60) to operate the restriction device (4, 56).

54. The apparatus according to claim 53, wherein the operation device (60) comprises hydraulic means and at least one valve for controlling a fluid flow in the hydraulic means.

55. The apparatus according to claim 54, wherein the control device (6, 10) comprises a wireless remote control for controlling the valve.

56. The apparatus according to claim 53, wherein the restriction device (4, 56) comprises hydraulic means and the operation device (60) comprises a reservoir (20, 34, 46, 230) forming a fluid chamber with a variable volume connected to the hydraulic means, and the operation device (60) is adapted to distribute fluid from the chamber to the hydraulic means by reduction of the volume of the chamber and to withdraw fluid from the hydraulic means to the chamber by expansion of the volume of the chamber.

57. The apparatus according to any of claims 8,52 and 53, wherein the operation device (60) comprises the motor (16, 18, 36, 232).

58. The apparatus according to claim 57, wherein the motor (16, 18, 36, 232) comprises a rotary motor, and the control device (6, 10) controls the rotary motor to rotate a desired number of revolutions.

59. The apparatus according to claim 57, wherein the motor (16, 18, 36, 232) comprises a linear motor.

60. The apparatus according to claim 57, wherein the motor (16, 18, 36, 232) comprises a hydraulic or pneumatic fluid motor, and the control device (6, 10) controls the fluid motor.

61. The apparatus according to claim 57, wherein the motor (16, 18, 36, 232) comprises an electric motor having electrically conductive parts made of plastics.

62. The apparatus according to any one of claims 1, 45-53, wherein the control device (6, 10) releases polarized energy from the source of energy.

63. The apparatus according to any one of claims 45-53, wherein the control device (6, 10) shifts polarity of the released energy to reverse the operation device (60).

64. The apparatus according to any one of claims 45-53, wherein the operation device (60) comprises an electric motor and the released energy comprises electric energy.

65. The apparatus according to any one of claims 1, 8, 23, 52, 53 and 57, wherein the restriction device (4, 56) is operable to perform a reversible function.

66. The apparatus according to claim 65, further comprising a reversing device (14, 38, 52, 234) implantable in the patient for reversing the function performed by the restriction device (4, 56).

67. The apparatus according to claim 66, wherein the control device (6, 10) controls the reversing device (14, 38, 52, 234) to reverse the function performed by the restriction device (4, 56).

68. The apparatus according to claim 66, wherein the reversing device (14, 38, 52, 234) comprises hydraulic means including a valve for shifting the flow direction of a fluid in the hydraulic means.

69. The apparatus according to claim 66, wherein the reversing device comprises a mechanical reversing device (14, 52).

70. The apparatus according to claim 69, wherein the mechanical reversing device comprises a switch (14).

71. The apparatus according to claim 69, wherein the reversing device comprises a gearbox (52).

72. The apparatus according to claim 66, wherein the reversing device comprises a switch (14).

73. The apparatus according to claim 72, wherein the switch of the reversing device (14) is operable by the released energy.

74. The apparatus according to claim 73, wherein the control device (6, 10) controls the operation of the switch of the reversing device (14) by shifting polarity of the released energy supplied to the switch.

75. The apparatus according to claim 72, wherein the switch comprises an electric switch and the source of energy supplies electric energy for the operation of the switch.

76. The apparatus according to any of claims 1, 2, 8, 23 52, 53 and 57, wherein the restriction device (4, 56) comprises hydraulic means and the operation device (60) is adapted to conduct a hydraulic fluid in the hydraulic means.

77. The apparatus according to claim 76, wherein the operation device (60) comprises the motor (16, 18, 36, 232)

78. The apparatus according to claim 76 or 77, wherein the operation device (60) comprises a fluid conduit connected to the hydraulic means of the restriction device (4, 56), and a reservoir (20, 34, 46, 230) for hydraulic fluid, the reservoir (20, 34, 46, 230) forming part of the conduit.

79. The apparatus according to claim 78, wherein the hydraulic means and conduit is devoid of any non-return valve.

80. The apparatus according to claim 79, wherein the reservoir (20, 34, 46, 230) forms a fluid chamber with a variable volume, and the operation device (60) is adapted to distribute fluid from the chamber to the hydraulic means of the restriction device (4, 56) by reduction of the volume of the chamber and to withdraw fluid from the hydraulic means to the chamber by expansion of the volume of the chamber.

81. The apparatus according to any of the preceding claims, further comprising at least one implantable sensor (54) for sensing at least one physical parameter of the patient.

82. The apparatus according to claim 81, wherein the sensor (54) comprises a pressure sensor for directly or indirectly sensing as the physical parameter the pressure in the urethra or urine bladder (58).

83. The apparatus according to claim 81 or 82, wherein the control device (6, 10) controls the restriction device (4, 56) in response to signals from the sensor (54).

84. The apparatus according to claim 83, wherein the control device (6, 10) comprises an internal control unit (138) implantable in the patient, the internal control unit (138) controlling the restriction device (4, 56) in response to signals from the sensor (54).

85. The apparatus according to claim 84, wherein the control device (6, 10) comprises an external control unit (136) outside the patient's body, the external control unit (136) controlling the restriction device (4, 56) in response to signals from the sensor (54).

86. The apparatus according to claim 85, wherein the external control unit (136) stores information on the physical parameter sensed by the sensor (54) and is manually operated to control the restriction device (4, 56) based on the stored information.

87. The apparatus according to any of claims 81-86, further comprising at least one implantable sender for sending information on the physical parameter sensed by the sensor (54).

88. The apparatus according to any of the preceding claims, wherein the external data communicator feeds data to the internal data communicator.

89. The apparatus according to claim 88, wherein the internal data communicator feeds data related to the restriction device (4, 56).

90. The apparatus according to claim 88 or 89, wherein the implantable communicator feeds data related to at least one physical signal of the patient.

91. The apparatus according to any of the preceding claims, wherein the restriction device (4, 56) is adapted to control the restriction of the urine passageway.

92. The apparatus according to claim 91, wherein the restriction device is operable to enlarge and restrict the urine passageway when implanted in the patient.

93. The apparatus according to claim 91 or 92, wherein the restriction device (4, 56) is adapted to steplessly control the restriction of the urine passageway when implanted in the patient.

94. The apparatus according to any of the preceding claims, further comprising an implantable energy transforming device (6, 62), wherein the control device (6, 10) is adapted to control the source of energy to release wireless electric energy, and the energy transforming device (6, 62) is adapted to transform the electric energy into kinetic energy for operation of the restriction device (4, 56).

95. The apparatus according to claim 94, wherein the restriction device is directly operated with the kinetic energy, as the energy transforming device (6, 62) transforms the electric energy into the kinetic energy.

96. The apparatus according to any of the preceding claims, wherein the control device (6, 10) controls the source of energy to release magnetic energy, non-magnetic energy, electromagnetic energy, non-electromagnetic energy, kinetic energy, non-kinetic energy, sonic energy, non-sonic energy, thermal energy or non-thermal energy.

97. The apparatus according to any of the preceding claims, wherein the restriction device (4, 56) is non-inflatable.

98. The apparatus according to any of the preceding claims, wherein the control device (6, 10) controls the source of energy to release energy for a determined time period.

99. The apparatus according to any of claims 1-97, wherein the control device (6, 10) controls the source of energy to release energy in a determined number of energy pulses.

100. The apparatus according to any of the preceding claims, wherein the control device (6, 10) is adapted to control the source of energy to release energy in a non-invasive manner.

101. The apparatus according to any of the preceding claims, wherein the control device (6, 10) comprises a wireless remote control for transmitting at least one wireless control signal for controlling the restriction device (4, 56).

102. The apparatus according to claim 101, wherein the remote control is capable of obtaining information on the condition of the restriction device (4, 56) when the restriction device (4, 56) is implantable and to control the restriction device (4, 56) in response to the information.

103. The apparatus according to claim 101 or 102, wherein the wireless remote control comprises at least one external signal transmitter or transceiver and at least one internal signal receiver or transceiver implantable in the patient.

104. The apparatus according to claim 101 or 102, wherein the wireless remote control comprises at least one external signal receiver or transceiver and at least one internal signal transmitter or transceiver implantable in the patient.

105. The apparatus according to any of claims 101-104, wherein the remote control is capable of sending information related to the restriction device (4, 56) from inside the patient's body to the outside thereof.

106. The apparatus according to claim 105, wherein the remote control controls the restriction device (4, 56) in response to the information.

107. The apparatus according to any of claims 102-106, wherein the remote control transmits a carrier signal for carrying the control signal.

108. The apparatus according to claim 107, wherein the carrier signal is frequency, amplitude or frequency and amplitude modulated.

109. The apparatus according to claim 107 or 108, wherein the carrier signal is digital, analog or digital and analog.

110. The apparatus according to any of claims 107-109, wherein the control signal used with the carrier signal is frequency, amplitude or frequency and amplitude modulated.

111. The apparatus according to any of claims 101-110, wherein the control signal comprises a wave signal comprising one of a sound wave signal including an ultrasound wave signal, an electromagnetic wave signal including an infrared light signal, a visible light signal, an ultra violet light signal and a laser light signal, a micro wave signal, a radio wave signal, an x-ray radiation signal, and a gamma radiation signal.

112. The apparatus according to any of claims 101-110, wherein the control signal comprises an electric, magnetic or electric and magnetic field.

113. The apparatus according to any of claims 101-111, wherein the control signal is digital, analog or digital and analog.

114. The apparatus according to claim 113, wherein the remote control transmits an electromagnetic carrier wave signal for carrying the digital or analog control signal.

115. The apparatus according to any of claims 101-114, wherein the control signal is transmitted in pulses by the wireless remote control.

116. The apparatus according to claim 2 or 3, further comprising an implantable stabilizer for stabilizing the energy released by the control device (6, 10).

117. The apparatus according to claim 116, wherein the energy released by the control device (6, 10) comprises electric energy and the stabilizer comprises at least one capacitor capacitor (162).

118. The apparatus according to claim 1, wherein the control device (6, 10) is adapted to control the source of energy to release energy for direct use in connection with the operation of the restriction device (4, 56).

119. The apparatus according to claim 1, wherein the control device (6, 10) is adapted to control the source of energy to intermittently release energy in the form of a train of energy pulses for direct use in connection with the operation of the restriction device (4, 56).

120. The apparatus according to claim 4 or 119, wherein the control device (6, 10) is adapted to control the source of energy to release electric energy, and further comprising an implantable capacitor for producing the train of energy pulses from the released energy.

121. The apparatus according to claim 1, further comprising implantable electrical components including at least one voltage level guard.

122. The apparatus according to claim 1, further comprising implantable electrical components including a single voltage level guard.

123. The apparatus according to claim 121 or 122, wherein the electrical components are devoid of any current detector and/or charge level detector.

124. The apparatus according to any of claims 120-123, further comprising an implantable capacitor or accumulator, wherein the charge or discharge of the capacitor or accumulator is controlled by use of the voltage level guard.

125. The apparatus according to claim 35, wherein the control device (6, 10) controls the external source of energy to release the wireless energy.

126. The apparatus according to claim 116, wherein the released energy comprises electric energy and further comprising an implantable capacitor for producing the train of energy pulses.

127. The apparatus according to claim 116 or 124, wherein the capacitor has a capacity less than 0,1 µF.

128. The apparatus according to any one of claims 1-3 and 116, wherein the control device (6, 10) is adapted to control the source of energy to directly power the motor (16, 18, 36, 232) with the released energy.

129. The apparatus according to claim 23, wherein the wireless energy comprises electromagnetic waves excluding radio waves.

130. The apparatus according to any of claims 2, 3 and 118, wherein the control device (6, 10) is adapted to release wireless energy in the form of a magnetic field or electromagnetic waves for direct power of the motor (16, 18, 36, 232), as the wireless energy is being released.

131. The apparatus according to any of claims 2-4, wherein the wireless energy comprises a signal.

132. The apparatus according to claim 1, further comprising an implantable energy transforming device (6, 62) for transforming wireless energy directly or indirectly into energy different than the wireless energy for operation of the restriction device (4, 56).

133. The apparatus according to claim 132, wherein the energy transforming device (6, 62) transforms the wireless energy in the form of sound waves into electric energy for operation of the restriction device.

134. The apparatus according to claim 133, wherein the energy transforming device (6, 62) transforms the wireless energy in the form of sound waves directly into electric energy.

135. The apparatus according to any of claims 133 or 134, wherein the energy transforming device (6, 62) comprises a capacitor.

136. The apparatus according to claim 135, wherein the capacitor is adapted to produce electric pulses from the transformed electric energy.

137. The apparatus according to any of claims 132-136, wherein the motor (16, 18, 36, 232) is powered by the transformed energy.

138. The apparatus according to claim 1, wherein the restriction device (4, 56) is adjustable in a non-manual manner.

139. The apparatus according to any of the preceding claims, wherein the restriction device (4, 56) is embedded in a soft or gel-like material.

140. The apparatus according to any of the preceding claims, wherein the restriction device (4, 56) is embedded in a silicone material having hardness less than 20 Shore.

141. The apparatus according to claim 3, further comprising an activatable source of energy implantable in the patient, wherein the implantable source of energy is activated by wireless energy released from the external source of energy, to supply energy which is used in connection with the operation of the restriction device (4, 56).

142. The apparatus according to any one of the preceding claims, wherein the control device (6, 10) is adapted to control the motor or pump (16, 18, 36, 232) by releasing energy to operate the motor or pump to operate the restriction device (4, 56) to both restrict the urine passageway and reverse the function performed by the restriction device (4, 56).

## Patentansprüche

1. Vorrichtung zur Behandlung von Harninkontinenz, umfassend:
- eine Restriktionseinrichtung (4, 56) für ein Angreifen an der Harnröhre oder Harnblase (58), um einen eingeschränkten Harndurchlass in der Harnröhre oder Harnblase (58) zu bilden, wobei die Restriktionseinrichtung so betätigbar ist, dass sie die Restriktion des Harndurchlasses ändert,
- eine Energiequelle (32, 42, 236) und
- eine Steuerungseinrichtung (6, 10), die von außerhalb des Körpers des Patienten betätigbar ist, um die Energiequelle so zu steuern, dass sie Energie zur Verwendung im Zusammenhang mit der Betätigung der Restriktionseinrichtung (4, 56) abgibt, wobei die Vorrichtung umfasst:
- einen in den Patienten implantierbaren Motor oder eine in den Patienten implantierbare Pumpe (16, 18, 36, 232), wobei die Energiequelle (32, 42, 236) dafür vorgesehen ist, den Motor oder die Pumpe (16, 18, 36, 232) anzutreiben, und die Steuerungseinrichtung (6, 10) dafür vorgesehen ist, den Motor oder die Pumpe (16, 18, 36, 232) so zu steuern, dass die Restriktionseinrichtung (4, 56) betätigt wird,
**dadurch gekennzeichnet, dass** die Vorrichtung umfasst:
- eine externe Datenkommunikationseinrichtung, die dafür vorgesehen ist, sich außerhalb des Körpers des Patienten zu befinden, und
- eine in den Patienten implantierbare interne Datenkommunikationseinrichtung für eine Kommunikation mit der externen Kommunikationseinrichtung, wobei die interne Datenkommunikationseinrichtung patientenbezogene Daten zurück an die externe Datenkommunikationseinrichtung leitet.

2. Vorrichtung nach Anspruch 1, bei der die Energiequelle dafür vorgesehen ist, sich außerhalb des Körpers des Patienten zu befinden, wenn die Restriktionseinrichtung (4, 56) darin implantiert ist, und die Steuerungseinrichtung (6, 10) dafür vorgesehen ist, die externe Energiequelle so zu steuern, dass sie für eine Verwendung im Zusammenhang mit der Betätigung der Restriktionseinrichtung (4, 56) drahtlose Energie abgibt.

3. Vorrichtung nach Anspruch 2, bei der die Steuerungseinrichtung (6, 10) dafür vorgesehen ist, die externe Energiequelle so zu steuern, dass sie für eine direkte Nutzung im Zusammenhang mit der Betätigung der Restriktionseinrichtung (4, 56) drahtlose Energie abgibt.

4. Vorrichtung nach Anspruch 2 oder 3, bei der die Steuerungseinrichtung (6, 10) dafür vorgesehen ist, die externe Energiequelle so zu steuern, dass sie in Intervallen drahtlose Energie in Form einer Folge von Energieimpulsen für eine direkte Verwendung im Zusammenhang mit der Betätigung der Restriktionseinrichtung (4, 56) abgibt.

5. Vorrichtung nach einem der Ansprüche 2-4, bei der die Restriktionseinrichtung (4, 56) auf nicht magnetische, nicht thermische, nicht manuelle oder nicht mechanische Art durch Verwendung der abgegebenen drahtlosen Energie betätigbar ist.

6. Vorrichtung nach Anspruch 1, bei der die Restriktionseinrichtung (4, 56) durch die abgegebene Energie auf manuelle, mechanische, thermische oder magnetische Art betätigbar ist.

7. Vorrichtung nach Anspruch 1, bei der die Restriktionseinrichtung (4, 56) durch die abgegebene Energie auf nicht manuelle, nicht mechanische, nicht thermische oder nicht magnetische Art betätigbar ist.

8. Vorrichtung nach Anspruch 1 oder 2, weiterhin umfassend eine in den Patienten implantierbare Betätigungseinrichtung (60), die dafür vorgesehen ist, die Rcstriktionseinrichtung (4, 56) zu betätigen.

9. Vorrichtung nach Anspruch 8, bei der die Betätigungseinrichtung (60) durch magnetische Energie, nicht magnetische Energie, elektromagnetische Energie, nicht elektromagnetische Energie, kinetische Energie, nicht kinetische Energie, thermische Energie oder nicht thermische Energie angetrieben wird.

10. Vorrichtung nach Anspruch 8, bei der die Betätigungseinrichtung (60) eine elektrische Betätigungseinrichtung (60) umfasst.

11. Vorrichtung nach einem der Ansprüche 1 oder 2, bei der die Steuerungseinrichtung (6, 10) auf manuelle oder nicht manuelle Art aktiviert wird, um die Energiequelle so zu steuern, dass Energie abgegeben wird.

12. Vorrichtung nach Anspruch 1, weiterhin umfassend eine Einstelleinrichtung zum Einstellen der Restriktionseinrichtung (4, 56) zum Ändern der Restriktion des Harndurchlasses, wobei die Einstelleinrichtung dafür vorgesehen ist, die Restriktionseinrichtung (4, 56) mechanisch einzustellen, oder dafür vorgesehen ist, die Restriktionseinrichtung (4, 56) unter Verwendung eines hydraulischen Mittels hydraulisch einzustellen, das kein hydraulisches Fluid der Art aufweist, das eine Viskosität hat, die bei Einwirkung von Wärme oder eines Magnetfelds wesentlich ansteigt.

13. Vorrichtung nach einem der Ansprüche 1-12, bei der die Steuerungseinrichtung (6, 10) dafür vorgesehen ist, die Restriktionseinrichtung (4, 56) zu steuern.

14. Vorrichtung nach Anspruch 13, bei der die Steuerungseinrichtung (6, 10) eine in den Patienten implantierbare interne Steuerungseinheit (138) zur Steuerung der Restriktionseinrichtung (4, 56) umfasst.

15. Vorrichtung nach Anspruch 14, bei der die interne Steuerungseinheit (138) programmierbar ist.

16. Vorrichtung nach Anspruch 15, bei der die Steuerungseinrichtung (6, 10) eine externe Steuerungseinheit (136) umfasst, die dafür vorgesehen ist, sich außerhalb des Körpers des Patienten zu befinden, wobei die interne Steuerungseinheit (138) durch die externe Steuerungseinheit (136) programmierbar ist.

17. Vorrichtung nach Anspruch 15, bei der die interne Steuerungseinheit (138) so programmierbar ist, dass sie die Restriktionseinrichtung (4, 56) im Verlauf der Zeit steuert.

18. Vorrichtung nach Anspruch 17, bei der die interne Steuerungseinheit (138) dafür vorgesehen ist, die Restriktionseinzichtung (4, 56) im Verlauf der Zeit entsprechend einem Aktivitäts-Zeitplan zu steuern.

19. Vorrichtung nach Anspruch 17, bei der die interne Steuerungseinheit (138) einen Mikroprozessor (176) umfasst.

20. Vorrichtung nach Anspruch 16, bei der die externe Steuerungseinheit (136) die interne Steuerungseinheit (138) entsprechend einem Lademodus, der nur für einen Arzt zugelassen ist, mit Daten lädt.

21. Vorrichtung nach Anspruch 16, bei dem die externe Steuerungseinheit (136) dafür vorgesehen ist, die interne Steuerungseinheit (138) entsprechend einem Arzt-Modus, der nur für einen Arzt zugelassen ist, zu steuern.

22. Vorrichtung nach Anspruch 16, bei der die externe Steuerungseinheit (136) dafür vorgesehen ist, die interne Steuerungseinheit (138) entsprechend einem Patientenmodus, der für den Patienten zugelassen ist, zu steuern.

23. Vorrichtung nach Anspruch 1, bei der die Energiequelle in den Patienten implantierbar ist.

24. Vorrichtung nach Anspruch 23, bei der die implantierbare Energiequelle wenigstens einen Akkumulator, wenigstens einen Kondensator oder wenigstens eine aufladbare Batterie oder eine Kombination aus wenigstens einem Kondensator und wenigstens einer aufladbaren Batterie umfasst.

25. Vorrichtung nach Anspruch 24, bei der die implantierbare Energiequelle eine elektrische Energiequelle umfasst.

26. Vorrichtung nach Anspruch 25, bei der die elektrische Energiequelle einen Akkumulator oder eine Batterie mit einer Lebensdauer von wenigstens 10 Jahren umfasst.

27. Vorrichtung nach Anspruch 2, die weiterhin eine in den Patienten implantierbare Energiespeichereinrichtung zum Speichern der drahtlosen Energie, die von der externen Energiequelle abgegeben wird, umfasst.

28. Vorrichtung nach Anspruch 27, bei der die Energiespeichereinrichtung einen Akkumulator umfasst.

29. Vorrichtung nach Anspruch 28, bei der der Akkumulator einen elektrischen Akkumulator umfasst.

30. Vorrichtung nach Anspruch 29, bei der der elektrische Akkumulator wenigstens einen Kondensator oder wenigstens eine aufladbare Batterie oder eine Kombination aus wenigstens einem Kondensator und wenigstens einer aufladbaren Batterie umfasst.

31. Vorrichtung nach Anspruch 2, die weiterhin eine in den Patienten implantierbare Batterie umfasst, um elektrische Energie an implantierbare, elektrische Energie verbrauchende Bauteile der Vorrichtung zu liefern.

32. Vorrichtung nach einem der Ansprüche 1, 2, 13 und 23, die weiterhin einen in den Patienten implantierbaren Schalter (238) umfasst, um die Betätigung der Restriktionseinrichtung (4, 56) direkt oder indirekt umzuschalten.

33. Vorrichtung nach Anspruch 32, die weiterhin eine in den Patienten implantierbare interne Energiequelle umfasst, um Energie für die Betätigung der Restriktionseinrichtung (4, 56) zu liefern, wobei der Schalter (238) die Zufuhr von Energie von der internen Energiequelle direkt oder indirekt beeinflusst.

34. Vorrichtung nach Anspruch 33, bei der der Schalter (238) dafür vorgesehen ist, zwischen einem "Aus"-Modus, bei dem die interne Energiequelle nicht in Gebrauch ist, und einem "Ein"-Modus, bei dem die interne Energiequelle Energie für die Betätigung der Restriktionseinrichtung (4, 56) liefert, umzuschalten.

35. Vorrichtung nach Anspruch 34, bei der der Schalter (238) durch die drahtlose Energie betätigbar ist, die von der externen Energiequelle abgegeben wird.

36. Vorrichtung nach einem der Ansprüche 1, 2 und 35, bei der die Steuerungseinrichtung (6, 10) eine drahtlose Fernsteuerung umfasst.

37. Vorrichtung nach Anspruch 33, bei der die Steuerungseinrichtung (6, 10) eine drahtlose Fernsteuerung zum Steuern der internen Energiequelle umfasst.

38. Vorrichtung nach Anspruch 37, bei der der Schalter (238) durch die drahtlose Energie von der externen Energiequelle so betätigbar ist, dass er zwischen einem "Aus"-Modus, bei dem die interne Energiequelle und die Fernsteuerung nicht in Gebrauch sind, und einem "Bereitschafts"-Modus, bei dem die Fernsteuerung die interne Energiequelle steuern kann, um Energie für die Betätigung der Restriktionseinrichtung (4, 56) zu liefern, umschalten kann.

39. Vorrichtung nach Anspruch 33, die weiterhin eine in den Patienten implantierbare Energieumwandlungseinheit (6, 62) zum Umwandeln der drahtlosen Energie in speicherbare Energie sowie eine in den Patienten implantierbare Energiespeichereinrichtung zum Speichern der speicherbaren Energie umfasst.

40. Vorrichtung nach Anspruch 39, bei der der Schalter (238) durch Energie von der implantierbaren Energiespeichereinrichtung so betätigbar ist, dass er zwischen einem "Aus"-Modus, bei dem die interne Energiequelle nicht in Gebrauch ist, und einem "Ein"-Modus, bei dem die interne Energiequelle Energie für den Betrieb der Restriktionseinrichtung (4, 56) liefert, umschaltet.

41. Vorrichtung nach Anspruch 40, bei der die Steuerungseinrichtung (6, 10) dafür vorgesehen ist, die Energiespeichereinrichtung so zu steuern, dass sie den Schalter (238) betätigt.

42. Vorrichtung nach Anspruch 13 oder 41, bei der die Steuerungseinrichtung (6, 10) eine drahtlose Fernsteuerung umfasst.

43. Vorrichtung nach Anspruch 33, die weiterhin eine in den Patienten implantierbare Energieumwandlungseinrichtung (6, 62) umfasst, um die drahtlose Energie in spcicherbare Energie umzuwandeln, wobei die interne Energiequelle in der Lage ist, die speicherbare Energie zu speichern.

44. Vorrichtung nach Anspruch 43, bei der der Schalter (238) von einem "Aus"-Modus, bei dem die interne Energiequelle nicht in Gebrauch ist, in einen "Ein"-Modus umschaltet, bei dem die Energiequelle Energie für die Betätigung der Restriktionseinrichtung (4, 56) liefert.

45. Vorrichtung nach Anspruch 44, bei der die Steuerungseinrichtung (6, 10) dafür vorgesehen ist, den Schalter (238) so zu steuern, dass er zwischen dem "Ein"- und dem "Aus"-Modus umschaltet.

46. Vorrichtung nach Anspruch 45, bei der die Steuerungseinrichtung (6, 10) eine drahtlose Fernsteuerung umfasst.

47. Vorrichtung nach Anspruch 33, bei der die interne Energiequelle eine elektrische Energiequelle umfasst.

48. Vorrichtung nach Anspruch 47, bei der die elektrische Energiequelle wenigstens einen Akkumulator, wenigstens einen Kondensator oder wenigstens eine aufladbare Batterie oder eine Kombination aus wenigstens einem Kondensator und wenigstens einer aufladbaren Batterie umfasst.

49. Vorrichtung nach Anspruch 47, bei der die elektrische Energiequelle einen Akkumulator oder eine Batterie mit einer Lebensdauer von wenigstens 10 Jahren umfasst.

50. Vorrichtung nach Anspruch 1, bei der die Energiequelle eine nukleare Energiequelle umfasst.

51. Vorrichtung nach Anspruch 1, bei der die Energiequelle eine chemische Energiequelle umfasst.

52. Vorrichtung nach Anspruch 3, die weiterhin eine in den Patienten implantierbare Betätigungseinrichtung (60) zum Betätigen der Restriktionseinrichtung (4, 56) umfasst, wobei die drahtlose Energie die Betätigungseinrichtung (60) direkt oder indirekt antreibt.

53. Vorrichtung nach Anspruch 8 oder 52, bei der die Steuerungseinrichtung (6, 10) dafür vorgesehen ist, die Betätigungseinrichtung (60) so zu steuern, dass sie die Restriktionseinrichtung (4, 56) betätigt.

54. Vorrichtung nach Anspruch 53, bei der die Betätigungseinrichtung (60) ein hydraulisches Mittel und wenigstens ein Ventil zur Steuerung eines Fluidstroms in dem hydraulischen Mittel aufweist.

55. Vorrichtung nach Anspruch 54, bei der die Steuerungseinrichtung (6, 10) eine drahtlose Fernsteuerung zur Steuerung des Ventils aufweist.

56. Vorrichtung nach Anspruch 53, bei der die Restriktionseinrichtung (4, 56) ein hydraulisches Mittel umfasst und die Betätigungseinrichtung (60) einen Behälter (20, 34, 46, 230) umfasst, der eine Fluidkammer mit variablem Volumen bildet, die mit dem hydraulischen Mittel verbunden ist, und die Betätigungseinrichtung (60) dafür vorgesehen ist, Fluid von der Kammer zu dem hydraulischen Mittel zu leiten, indem das Volumen der Kammer reduziert wird, und Fluid von dem hydraulischen Mittel zu der Kammer abzuziehen, indem das Volumen der Kammer erhöht wird.

57. Vorrichtung nach einem der Ansprüche 8, 52 und 53, bei der die Betätigungseinrichtung (60) den Motor (16, 18, 36, 232) umfasst.

58. Vorrichtung nach Anspruch 57, bei der der Motor (16, 18, 36, 232) einen Drehmotor umfasst und die Steuerungseinrichtung (6, 10) den Drehmotor so steuert, dass er eine gewünschte Zahl von Umdrehungen ausführt.

59. Vorrichtung nach Anspruch 57, bei der der Motor (16, 18, 36, 232) einen Linearmotor umfasst.

60. Vorrichtung nach Anspruch 57, bei der der Motor (16, 18, 36, 232) einen hydraulischen oder pneumatischen Fluidmotor umfasst und die Steuerungseinrichtung (6, 10) den Fluidmotor steuert.

61. Vorrichtung nach Anspruch 57, bei der der Motor (16, 18, 36, 232) einen Elektromotor mit aus Kunststoff hergestellten elektrisch leitenden Teilen umfasst.

62. Vorrichtung nach einem der Ansprüche 1, 45-53, bei der die Steuerungseinrichtung (6, 10) polarisierte Energie von der Energiequelle abgibt.

63. Vorrichtung nach einem der Ansprüche 45-53, bei der die Steuerungseinrichtung (6, 10) die Polarität der abgegebenen Energie umkehrt, um die Betätigungseinrichtung (60) umzukehren.

64. Vorrichtung nach einem der Ansprüche 45-53, bei der die Betätigungseinrichtung (60) einen Elektromotor umfasst und die abgegebene Energie elektrische Energie umfasst.

65. Vorrichtung nach einem der Ansprüche 1, 8, 23, 52, 53 und 57, bei der die Restriktionseinrichtung (4, 56) so betätigbar ist, dass sie eine umkehrbare Funktion ausführt.

66. Vorrichtung nach Anspruch 65, die weiterhin eine in den Patienten implantierbare Umkehreinrichtung (14, 38, 52, 234) umfasst, um die von der Restriktionseinrichtung (4, 56) ausgeübte Funktion umzukehren.

67. Vorrichtung nach Anspruch 66, bei der die Steuerungseinrichtung (6, 10) die Umkehreinrichtung (14, 38, 52, 234) steuert, um die von der Restriktionseinrichtung (4, 56) ausgeübte Funktion umzukehren.

68. Vorrichtung nach Anspruch 66, bei der die Umkehreinrichtung (14, 38, 52, 234) ein hydraulisches Mittel umfasst, das ein Ventil aufweist, um die Strömungsrichtung eines Fluids in dem hydraulischen Mittel umzukehren.

69. Vorrichtung nach Anspruch 66, bei der die Umkehreinrichtung eine mechanische Umkehreinrichtung (14, 52) umfasst.

70. Vorrichtung nach Anspruch 69, bei der die mechanische Umkehreinrichtung einen Schalter (14) aufweist.

71. Vorrichtung nach Anspruch 69, bei der die Umkehreinrichtung ein Getriebe (52) umfasst.

72. Vorrichtung nach Anspruch 66, bei der die Umkehreinrichtung einen Schalter (14) aufweist.

73. Vorrichtung nach Anspruch 72, bei der der Schalter (14) der Umkehreinrichtung von der abgegebenen Energie betätigbar ist.

74. Vorrichtung nach Anspruch 73, bei der die Steuerungseinrichtung (6, 10) die Betätigung des Schalters (14) der Umkehreinrichtung steuert, indem die Polarität der dem Schalter zugeführten abgegebenen Energie umgekehrt wird.

75. Vorrichtung nach Anspruch 72, bei der der Schalter einen elektrischen Schalter umfasst und die Energiequelle elektrische Energie für die Betätigung des Schalters liefert.

76. Vorrichtung nach einem der Ansprüche 1, 2, 8, 23, 52, 53 und 57, bei der die Restriktionseinrichtung (4, 56) ein hydraulisches Mittel umfasst und die Betätigungseinrichtung (60) dafür vorgesehen ist, ein hydraulisches Fluid in dem hydraulischen Mittel zu leiten.

77. Vorrichtung nach Anspruch 76, bei der die Betätigungseinrichtung (60) den Motor (16, 18, 36, 232) umfasst.

78. Vorrichtung nach Anspruch 76 oder 77, bei der die Betätigungseinrichtung (60) eine Fluidleitung, die mit dem hydraulischen Mittel der Restriktionseinrichtung (4, 56) verbunden ist, und einen Behälter (20, 34, 46, 230) für Hydraulikfluid umfasst, wobei der Behälter (20, 34, 46, 230) einen Teil der Leitung bildet.

79. Vorrichtung nach Anspruch 78, bei der das hydraulische Mittel und die Leitung kein Rückschlagventil aufweisen.

80. Vorrichtung nach Anspruch 79, bei der der Behälter (20, 34, 46, 230) eine Fluidkammer mit variablem Volumen bildet und die Betätigungseinrichtung (60) dafür vorgesehen ist, Fluid von der Kammer an das hydraulische Mittel der Restriktionseinrichtung (4, 56) zu leiten, indem das Volumen der Kammer reduziert wird, und Fluid von dem hydraulischen Mittel zu der Kammer abzuziehen, indem das Volumen der Kammer erhöht wird.

81. Vorrichtung nach einem der vorigen Ansprüche, die weiterhin wenigstens einen implantierbaren Sensor (54) zum Erfassen von wenigstens einem physischen Parameter des Patienten umfasst.

82. Vorrichtung nach Anspruch 81, bei der der Sensor (54) einen Drucksensor zum direkten oder indirekten Erfassen des Drucks in der Harnröhre oder Harnblase (58) als physischem Parameter umfasst.

83. Vorrichtung nach Anspruch 81 oder 82, bei der die Steuerungseinrichtung (6, 10) die Restriktionseinrichtung (4, 56) ansprechend auf Signale von dem Sensor (54) steuert.

84. Vorrichtung nach Anspruch 83, bei der die Steuerungseinrichtung (6, 10) eine in den Patienten implantierbare interne Steuerungseinrichtung (138) umfasst, wobei die interne Steuerungseinrichtung (138) die Restriktionseinrichtung (4, 56) ansprechend auf Signale von dem Sensor (54) steuert.

85. Vorrichtung nach Anspruch 84, bei der die Steuerungseinrichtung (6, 10) eine externe Steuerungseinheit (136) außerhalb des Körpers des Patienten umfasst, wobei die externe Steuerungseinheit (136) die Restriktionseinrichtung (4, 56) ansprechend auf Signale von dem Sensor (54) steuert.

86. Vorrichtung nach Anspruch 85, bei der die externe Steuerungseinheit (136) Informationen über den physischen Parameter speichert, der von dem Sensor (54) erfasst wird, und manuell betätigt wird, um die Restriktionseinrichtung (4, 56) auf der Grundlage der gespeicherten Informationen zu steuern.

87. Vorrichtung nach einem der Ansprüche 81-86, die weiterhin wenigstens einen implantierbaren Sender zum Senden von Informationen über den von dem Sensor (54) erfassten physischen Parameter umfasst.

88. Vorrichtung nach einem der vorigen Ansprüche, bei der die externe Datenkommunikationseinrichtung Daten an die interne Datenkommunikationseinrichtung überträgt.

89. Vorrichtung nach Anspruch 88, bei der die interne Datenkommunikationseinrichtung Daten überträgt, die sich auf die Restriktionseinrichtung (4, 56) beziehen.

90. Vorrichtung nach einem der Ansprüche 88 oder 89, bei der die implantierbare Kommunikationseinrichtung Daten überträgt, die sich auf wenigstens ein physisches Signal des Patienten beziehen.

91. Vorrichtung nach einem der vorigen Ansprüche, bei der die Restriktionseinrichtung (4, 56) dafür vorgesehen ist, die Restriktion des Harndurchlasses zu steuern.

92. Vorrichtung nach Anspruch 91, bei der die Restriktionseinrichtung so betätigbar ist, dass sie den Harndurchlass vergrößert und einschränkt, wenn sie in den Patienten implantiert ist.

93. Vorrichtung nach Anspruch 91 oder 92, bei der die Restriktionseinrichtung (4, 56) dafür vorgesehen ist, stufenlos die Restriktion des Harndurchlasses zu steuern, wenn sie in den Patienten implantiert ist.

94. Vorrichtung nach einem der vorigen Ansprüche, die weiterhin eine implantierbare Energieumwandlungseinrichtung (6, 62) umfasst, wobei die Steuerungseinrichtung (6, 10) dafür vorgesehen ist, die Energiequelle so zu steuern, dass sie drahtlose elektrische Energie abgibt, und die Energicumwandlungseinrichtung (6, 62) dafür vorgesehen ist, die elektrische Energie in kinetische Energie für die Betätigung der Restriktionseinrichtung (4, 56) umzuwandeln.

95. Vorrichtung nach Anspruch 94, bei der die Restriktionseinrichtung direkt mit der kinetischen Energie betätigt wird, wenn die Energieumwandlungseinrichtung (6, 62) die elektrische Energie in die kinetische Energie umwandelt.

96. Vorrichtung nach einem der vorigen Ansprüche, bei der die Steuerungseinrichtung (6, 10) die Energiequelle so steuert, dass sie magnetische Energie, nicht magnetische Energie, elektromagnetische Energie, nicht elektromagnetische Energie, kinetische Energie, nicht kinetische Energie, Schallenergie, Nicht-Schallenergie, Wärmeenergie oder Nicht-Wärmeenergie abgibt.

97. Vorrichtung nach einem der vorigen Ansprüche, bei der die Restriktionseinrichtung (4, 56) nicht aufblasbar ist.

98. Vorrichtung nach einem der vorigen Ansprüche, bei der die Steuerungseinrichtung (6, 10) die Energiequelle so steuert, dass sie eine bestimmte Zeit lang Energie abgibt.

99. Vorrichtung nach einem der Ansprüche 1-97, bei der die Steuerungseinrichtung (6, 10) die Energiequelle so steuert, dass sie Energie in einer bestimmten Zahl von Energieimpulsen abgibt.

100. Vorrichtung nach einem der vorigen Ansprüche, bei der die Steuerungseinrichtung (6, 10) dafür vorgesehen ist, die Energiequelle so zu steuern, dass sie Energie auf nicht invasive Art abgibt.

101. Vorrichtung nach einem der vorigen Ansprüche, bei der die Steuerungseinrichtung (6, 10) eine drahtlose Fernsteuerung umfasst, um wenigstens ein drahtloses Steuerungssignal zur Steuerung der Restriktionseinrichtung (4, 56) zu übertragen.

102. Vorrichtung nach Anspruch 101, bei der die Fernsteuerung in der Lage ist, Informationen über den Zustand der Restriktionseinrichtung (4, 56) zu erlangen, wenn die Restriktionseinrichtung (4, 56) implantierbar ist, und die Restriktionseinrichtung (4, 56) ansprechend auf die Informationen zu steuern.

103. Vorrichtung nach Anspruch 101 oder 102, bei der die drahtlose Fernsteuerung wenigstens einen externen Signaltransmitter oder -transceiver und wenigstens einen internen Signalempfänger oder -transeeiver umfasst, der in den Patienten implantierbar ist.

104. Vorrichtung nach Anspruch 101 oder 102, bei der die drahtlose Fernsteuerung wenigstens einen externen Signalempfänger oder -transceiver und wenigstens einen internen Signaltransmitter oder -transceiver umfasst, der in den Patienten implantierbar ist.

105. Vorrichtung nach einem der Ansprüche 101-104, bei der die Fernsteuerung in der Lage ist, Informationen bezüglich der Restriktionseinrichtung (4, 56) vom Inneren des Körpers des Patienten zu dessen Außenseite zu senden.

106. Vorrichtung nach Anspruch 105, bei der die Fernsteuerung die Restriktionseinrichtung (4, 56) ansprechend auf die Informationen steuert.

107. Vorrichtung nach einem der Ansprüche 102-106, bei der die Fernsteuerung ein Trägersignal zum Tragen des Steuerungssignals überträgt.

108. Vorrichtung nach Anspruch 107, bei der das Trägersignal frequenz-, amplituden- oder frequenz- und amplitudenmoduliert ist.

109. Vorrichtung nach Anspruch 107 oder 108, bei der das Trägersignal digital, analog oder digital und analog ist.

110. Vorrichtung nach einem der Ansprüche 107-109, bei der das mit dem Trägersignal verwendete Steuerungssignal frequenz-, amplituden- oder frequenz- und amplitudenmoduliert ist.

111. Vorrichtung nach einem der Ansprüche 101-110, bei der das Steuerungssignal ein Wellensignal umfasst, das eines der folgenden umfasst: ein Schallwellensignal, das ein Ultraschallwellensignal umfasst, ein elektromagnetisches Wellensignal, das ein Infrarotlichtsignal, ein Signal aus sichtbarem Licht, ein ultraviolettes Lichtsignal und ein Laserlichtsignal umfasst, ein Mikrowellensignal, ein Radiowellensignal, ein Röntgenstrahlungssignal und ein Gammastrahlungssignal.

112. Vorrichtung nach einem der Ansprüche 101-110, bei der das Steuerungssignal ein elektrisches, magnetisches oder elektrisches und magnetisches Feld umfasst.

113. Vorrichtung nach einem der Ansprüche 101-111, bei der das Steuerungssignal digital, analog oder digital und analog ist.

114. Vorrichtung nach Anspruch 113, bei der die Fernsteuerung ein elektromagnetisches Trägerwellensignal zum Tragen des digitalen oder analogen Steuerungssignals überträgt.

115. Vorrichtung nach einem der Ansprüche 101-114, bei der das Steuerungssignal in Impulsen durch die drahtlose Fernsteuerung übertragen wird.

116. Vorrichtung nach Anspruch 2 oder 3, die weiterhin einen implantierbaren Stabilisator zur Stabilisierung der Energie umfasst, die von der Steuerungseinrichtung (6, 10) abgegeben wird.

117. Vorrichtung nach Anspruch 116, bei der die von der Steuerungseinrichtung (6, 10) abgegebene Energie elektrische Energie umfasst und der Stabilisator wenigstens einen Kondensator (162) umfasst.

118. Vorrichtung nach Anspruch 1, bei der die Steuerungseinrichtung (6, 10) dafür vorgesehen ist, die Energiequelle so zu steuern, dass sie Energie für eine direkte Verwendung im Zusammenhang mit der Betätigung der Restriktionseinrichtung (4, 56) abgibt.

119. Vorrichtung nach Anspruch 1, bei der die Steuerungseinrichtung (6, 10) dafür vorgesehen ist, die Energiequelle so zu steuern, dass sie in Intervallen Energie in Form einer Energieimpulsfolge für eine direkte Verwendung im Zusammenhang mit der Betätigung der Restriktionseinrichtung (4, 56) abgibt.

120. Vorrichtung nach Anspruch 4 oder 119, bei der die Steuerungseinrichtung (6, 10) dafür vorgesehen ist, die Energiequelle so zu steuern, dass sie elektrische Energie abgibt, und die weiterhin einen implantierbaren Kondensator aufweist, um die Energieimpulsfolge aus der abgegebenen Energie zu erzeugen.

121. Vorrichtung nach Anspruch 1, die weiterhin implantierbare elektrische Bauteile umfasst, die wenigstens einen Spannungshöhenbegrenzer umfassen.

122. Vorrichtung nach Anspruch 1, die weiterhin implantierbare elektrische Bauteile umfasst, die einen Einzel-Spannungshöhenbegrenzer umfassen.

123. Vorrichtung nach einem der Ansprüche 121 oder 122, bei der die elektrischen Bauteile keine Stromstärkenerfassungseinrichtung und/oder Ladungshöhenerfassungseinrichtung aufweisen.

124. Vorrichtung nach einem der Ansprüche 120-123, die weiterhin einen implantierbaren Kondensator oder Akkumulator umfasst, wobei die Ladung oder Entladung des Kondensators oder Akkumulators durch Verwendung des Spannungshöhenbegrenzers gesteuert wird.

125. Vorrichtung nach Anspruch 35, bei der die Steuerungseinrichtung (6, 10) die externe Energiequelle so steuert, dass sie die drahtlose Energie abgibt.

126. Vorrichtung nach Anspruch 116, bei der die abgegebene Energie elektrische Energie umfasst, und die weiterhin einen implantierbaren Kondensator zur Erzeugung der Energieimpulsfolge umfasst.

127. Vorrichtung nach Anspruch 116 oder 124, bei der der Kondensator eine Kapazität von weniger als 0,1 µF hat.

128. Vorrichtung nach einem der Ansprüche 1-3 und 116, bei der die Steuerungseinrichtung (6, 10) dafür vorgesehen ist, die Energiequelle so zu steuern, dass sie den Motor (16, 18, 36, 232) mit der abgegebenen Energie direkt antreibt.

129. Vorrichtung nach Anspruch 23, bei der die drahtlose Energie elektromagnetische Wellen außer Radiowellen umfasst.

130. Vorrichtung nach einem der Ansprüche 2, 3 und 118, bei der die Steuerungseinrichtung (6, 10) dafür vorgesehen ist, drahtlose Energie in Form eines magnetischen Felds oder elektromagnetischer Wellen für einen direkten Antrieb des Motors (16, 18, 36, 232) abzugeben, wenn die drahtlose Energie abgegeben wird.

131. Vorrichtung nach einem der Ansprüche 2-4, bei der die drahtlose Energie ein Signal umfasst.

132. Vorrichtung nach Anspruch 1, die weiterhin eine implantierbare Energieumwandlungseinrichtung (6, 62) umfasst, um drahtlose Energie direkt oder indirekt in Energie umzuwandeln, die sich von der drahtlosen Energie für die Betätigung der Restriktionseinrichtung (4, 56) unterscheidet.

133. Vorrichtung nach Anspruch 132, bei der die Energieumwandlungseinrichtung (6, 62) die drahtlose Energie in Form von Schallwellen in elektrische Energie für die Betätigung der Restriktionseinrichtung umwandelt.

134. Vorrichtung nach Anspruch 133, bei der die Energieumwandlungseinrichtung (6, 62) die drahtlose Energie in Form von Schallwellen direkt in elektrische Energie umwandelt.

135. Vorrichtung nach einem der Ansprüche 133 oder 134, bei der die Energieumwandlungseinrichtung (6, 62) einen Kondensator umfasst.

136. Vorrichtung nach Anspruch 135, bei der der Kondensator dafür vorgesehen ist, elektrische Impulse aus der umgewandelten elektrischen Energie zu erzeugen.

137. Vorrichtung nach einem der Ansprüche 132-136, bei der der Motor (16, 18, 36, 232) von der umgewandelten Energie angetrieben wird.

138. Vorrichtung nach Anspruch 1, bei der die Restriktionseinrichtung (4, 56) auf nicht manuelle Art einstellbar ist.

139. Vorrichtung nach einem der vorigen Ansprüche, bei der die Restriktionseinrichtung (4, 56) in einem weichen oder gelartigen Material eingebettet ist.

140. Vorrichtung nach einem der vorigen Ansprüche, bei der die Restriktionseinrichtung (4, 56) in einem Silikonmaterial mit einer Shore-Härte von weniger als 20 eingebettet ist.

141. Vorrichtung nach Anspruch 3, die weiterhin eine aktivierbare Energiequelle umfasst, die in den Patienten implantierbar ist, wobei die implantierbare Energiequelle durch drahtlose Energie aktiviert wird, die von der externen Energiequelle (10, 40) abgegeben wird, um Energie zu liefern, die im Zusammenhang mit der Betätigung der Restriktionseinrichtung (4, 56) verwendet wird.

142. Vorrichtung nach einem der vorigen Ansprüche, bei der die Steuerungseinrichtung (6, 10) dafür vorgesehen ist, den Motor oder die Pumpe (16, 18, 36, 232) durch Abgeben von Energie zu steuern, um den Motor oder die Pumpe so zu betätigen, dass die Restriktionseinrichtung (4, 56) betätigt wird, um sowohl den Harndurchlass einzuschränken als auch die Funktion umzukehren, die von der Restriktionseinrichtung (4, 56) ausgeübt wird.

## Revendications

1. Appareil de traitement de l'incontinence urinaire, comprenant :
un dispositif de restriction (4, 56) pour engager l'urètre ou la vessie (58) pour former un passage d'urine restreint dans l'urètre ou la vessie (58), le dispositif de restriction pouvant être actionné pour modifier la restriction du passage d'urine,
une source d'énergie (32, 42, 236), et
un dispositif de commande (6, 10) pouvant être actionné depuis l'extérieur du corps du patient pour commander la source d'énergie pour libérer de l'énergie pour une utilisation en liaison avec l'actionnement du dispositif de restriction (4, 56), l'appareil comprenant :
un moteur ou une pompe (16, 18, 36, 232) pouvant être implanté(c) dans 1 patient, la source d'énergie (32, 42, 236) étant adaptée pour alimenter le moteur ou la pompe (16, 18, 36, 232), et le dispositif de commande (6, 10) est adapté pour commander le moteur ou la pompe (16, 18, 36, 232) pour actionner le dispositif de restriction (4, 56),
**caractérisé en ce que** l'appareil comprend :
un dispositif de communication de données externe conçu pour être à l'extérieur du corps du patient, et
un dispositif de communication de données interne pouvant être implanté dans le patient pour communiquer avec le dispositif de communication externe, le dispositif de communication de données interne communiquant des données liées au patient au dispositif de communication de données externe.

2. Appareil selon la revendication 1, dans lequel la source d'énergie est conçue pour être externe au corps du patient lorsque le dispositif de restriction (4, 56) est implanté à l'intérieur de celui-ci, et le dispositif de commande (6, 10) est adapté pour commander la source d'énergie externe pour libérer de l'énergie sans fil pour une utilisation en liaison avec l'actionnement du dispositif de restriction (4, 56).

3. Appareil selon la revendication 2, dans lequel le dispositif de commande (6, 10) est adapté pour commander la source d'énergie externe pour libérer de l'énergie sans fil pour une utilisation directe en liaison avec l'actionnement du dispositif de restriction (4, 56).

4. Appareil selon la revendication 2 ou 3, dans lequel le dispositif de commande (6, 10) est adapté pour commander la source d'énergie externe pour libérer par intermittence de l'énergie sans fil sous la forme d'un train d'impulsions d'énergie pour une utilisation directe en liaison avec l'actionnement du dispositif de restriction (4, 56).

5. Appareil selon l'une quelconque des revendications 2 à 4, dans lequel le dispositif de restriction (4, 56) peut être actionné de manière non magnétique, non thermique, non manuelle ou non mécanique par l'utilisation de ladite énergie sans fil libérée.

6. Appareil selon la revendication 1, dans lequel le dispositif de restriction (4, 56) peut être actionné par l'énergie libérée de manière manuelle, mécanique, thermique ou magnétique.

7. Appareil selon la revendication 1, dans lequel le dispositif de restriction (4, 56) peut être actionné par l'énergie libérée de manière non manuelle, non mécanique, non thermique ou non magnétique.

8. Appareil selon la revendication 1 ou 2, comprenant en outre un dispositif d'actionnement (60) pouvant être implanté dans le patient adapté pour actionner le dispositif de restriction (4, 56).

9. Appareil selon la revendication 8, dans lequel le dispositif d'actionnement (60) est alimenté par une énergie magnétique, une énergie non magnétique, une énergie électromagnétique, une énergie non électromagnétique, une énergie cinétique, une énergie non cinétique, une énergie thermique ou une énergie non thermique.

10. Appareil selon la revendication 8, dans lequel le dispositif d'actionnement (60) comprend un dispositif d'actionnement électrique (60).

11. Appareil selon la revendication 1 ou 2, dans lequel le dispositif de commande (6, 10) est activé de manière manuelle ou non manuelle pour commander la source d'énergie pour libérer de l'énergie.

12. Appareil selon la revendication 1, comprenant en outre un dispositif de réglage pour régler le dispositif de restriction (4, 56) pour modifier la restriction du passage d'urine, le dispositif de réglage étant adapté pour régler mécaniquement le dispositif de restriction (4, 56), ou adapté pour régler de manière hydraulique le dispositif de restriction (4, 56) en utilisant un moyen hydraulique qui est dépourvu de fluide hydraulique du type ayant une viscosité qui augmente sensiblement lorsqu'il est exposé à de la chaleur ou à un champ magnétique.

13. Appareil selon l'une quelconque des revendications 1 à 12, dans lequel le dispositif de commande (6, 10) est adapté pour commander le dispositif de restriction (4, 56).

14. Appareil selon la revendication 13, dans lequel le dispositif de commande (6, 10) comprend une unité de commande interne (138) pouvant être implantée dans le patient pour commander le dispositif de restriction (4, 56).

15. Appareil selon la revendication 14, dans lequel l'unité de commande interne (138) est programmable.

16. Appareil selon la revendication 15, dans lequel le dispositif de commande (6, 10) comprend une unité de commande externe (136) conçue pour être à l'extérieur du corps du patient, l'unité de commande interne (138) pouvant être programmée par l'unité de commande externe (136).

17. Appareil selon la revendication 15, dans lequel l'unité de commande interne (138) peut être programmée pour commander le dispositif de restriction (4, 56) dans le temps.

18. Appareil selon la revendication 17, dans lequel l'unité de commande interne (138) est adaptée pour commander le dispositif de restriction (4, 56) dans le temps en fonction d'un programme de planification d'activité.

19. Appareil selon la revendication 17, dans lequel l'unité de commande interne (138) comprend un microprocesseur (176).

20. Appareil selon la revendication 16, dans lequel l'unité de commande externe (136) charge l'unité de commande interne (138) avec des données en fonction d'un mode de chargement autorisé seulement pour un médecin.

21. Appareil selon la revendication 16, dans lequel l'unité de commande externe (136) est adaptée pour commander l'unité de commande interne (138) en fonction d'un mode médecin autorisé seulement pour un médecin.

22. Appareil selon la revendication 16, dans lequel l'unité de commande externe (136) est adaptée pour commander l'unité de commande interne (138) en fonction d'un mode patient permis pour le patient.

23. Appareil selon la revendication 1, dans lequel la source d'énergie peut être implantée dans le patient.

24. Appareil selon la revendication 23, dans lequel la source d'énergie pouvant être implantée comprend au moins un accumulateur, au moins un condensateur ou au moins une batterie rechargeable, ou une combinaison d'au moins un condensateur et au moins une batterie rechargeable.

25. Appareil selon la revendication 24, dans lequel la source d'énergie pouvant être implantée comprend une source d'énergie électrique.

26. Appareil selon la revendication 25, dans lequel la source d'énergie électrique comprend un accumulateur, ou une batterie ayant une durée de vie d'au moins 10 ans.

27. Appareil selon la revendication 2, comprenant en outre un dispositif de stockage d'énergie pouvant être implanté dans le patient pour stocker de l'énergie sans fil libérée par la source d'énergie externe.

28. Appareil selon la revendication 27, dans lequel le dispositif de stockage d'énergie comprend un accumulateur.

29. Appareil selon la revendication 28, dans lequel l'accumulateur comprend un accumulateur électrique.

30. Appareil selon la revendication 29, dans lequel l'accumulateur électrique comprend au moins un condensateur ou au moins une batterie rechargeable, ou une combinaison d'au moins un condensateur et au moins une batterie rechargeable.

31. Appareil selon la revendication 2, comprenant en outre une batterie pouvant être implantée dans le patient pour fournir de l'énergie électrique aux composants de l'appareil pouvant être implantés et consommant de l'énergie électrique.

32. Appareil selon l'une quelconque des revendications 1, 2, 13 et 23, comprenant en outre un commutateur (238) pouvant être implanté dans le patient pour commuter directement ou indirectement l'actionnement du dispositif de restriction (4, 56).

33. Appareil selon la revendication 32, comprenant en outre une source d'énergie interne pouvant être implantée dans le patient pour fournir de l'énergie pour l'actionnement du dispositif de restriction (4, 56), dans lequel le commutateur (238) affecte directement ou indirectement l'alimentation en énergie de la source d'énergie interne.

34. Appareil selon la revendication 33, dans lequel le commutateur (238) est adapté pour commuter entre un mode « désactivé », dans lequel la source d'énergie interne n'est pas utilisée, et un mode « activé », dans lequel la source d'énergie interne fournit de l'énergie pour l'actionnement du dispositif de restriction (4, 56).

35. Appareil selon la revendication 34, dans lequel le commutateur (238) peut être actionné par l'énergie sans fil libérée par la source d'énergie externe.

36. Appareil selon l'une quelconque des revendications 1, 2 et 35, dans lequel le dispositif de commande (6, 10) comprend une commande à distance sans fil.

37. Appareil selon la revendication 33, dans lequel le dispositif de commande (6, 10) comprend une commande à distance sans fil pour commander la source d'énergie interne.

38. Appareil selon la revendication 37, dans lequel le commutateur (238) peut être actionné par de l'énergie sans fil provenant de la source d'énergie externe pour commuter entre un mode « désactivé », dans lequel la source d'énergie interne et la commande à distance ne sont pas utilisées, et un mode « veille », dans lequel la commande à distance est permise pour commander la source d'énergie interne pour fournir de l'énergie pour l'actionnement du dispositif de restriction (4, 56).

39. Appareil selon la revendication 33, comprenant en outre un dispositif de transformation d'énergie (6, 62) pouvant être implanté dans le patient pour transformer l'énergie sans fil en énergie stockable et un dispositif de stockage d'énergie pouvant être implanté dans le patient pour stocker l'énergie stockable.

40. Appareil selon la revendication 39, dans lequel le commutateur (238) peut être actionné par de l'énergie provenant du dispositif de stockage d'énergie pouvant être implanté pour commuter entre un mode « désactivé », dans lequel la source d'énergie interne n'est pas utilisée, et un mode « activé », dans lequel la source d'énergie interne fournit de l'énergie pour l'actionnement du dispositif de restriction (4, 56).

41. Appareil selon la revendication 40, dans lequel le dispositif de commande (6, 10) est adapté pour commander le dispositif de stockage d'énergie pour actionner le commutateur (238).

42. Appareil selon la revendication 13 ou 41, dans lequel le dispositif de commande (6, 10) comprend une commande à distance sans fil.

43. Appareil selon la revendication 33, comprenant en outre un dispositif de transformation d'énergie (6, 62) pouvant être implanté dans le patient pour transformer l'énergie sans fil en énergie stockable, dans lequel la source d'énergie interne est capable de stocker l'énergie stockable.

44. Appareil selon la revendication 43, dans lequel le commutateur (238) commute d'un mode « désactivé », dans lequel la source d'énergie interne n'est pas utilisée, vers un mode « activé », dans lequel la source d'énergie fournit de l'énergie pour l'actionnement du dispositif de restriction (4, 56).

45. Appareil selon la revendication 44, dans lequel le dispositif de commande (6, 10) est adapté pour commander le commutateur (238) pour commuter entre les modes « activé » et « désactivé ».

46. Appareil selon la revendication 45, dans lequel le dispositif de commande (6, 10) comprend une commande à distance sans fil.

47. Appareil selon la revendication 33, dans lequel la source d'énergie interne comprend une source d'énergie électrique.

48. Appareil selon la revendication 47, dans lequel la source d'énergie électrique comprend au moins un accumulateur, au moins un condensateur ou au moins une batterie rechargeable, ou une combinaison d'au moins un condensateur et au moins une batterie rechargeable.

49. Appareil selon la revendication 47, dans lequel la source d'énergie électrique comprend un accumulateur ou une batterie ayant une durée de vie d'au moins 10 ans.

50. Appareil selon la revendication 1, dans lequel la source d'énergie comprend une source d'énergie nucléaire.

51. Appareil selon la revendication 1, dans lequel la source d'énergie comprend une source d'énergie chimique.

52. Appareil selon la revendication 3, comprenant en outre un dispositif d'actionnement (60) pouvant être implanté dans le patient pour actionner le dispositif de restriction (4, 56), dans lequel l'énergie sans fil alimente directement ou indirectement le dispositif d'actionnement (60).

53. Appareil selon la revendication 8 ou 52, dans lequel le dispositif de commande (6, 10) est adapté pour commander le dispositif d'actionnement (60) pour actionner le dispositif de restriction (4, 56).

54. Appareil selon la revendication 53, dans lequel le dispositif d'actionnement (60) comprend un moyen hydraulique et au moins une soupape pour commander un écoulement de fluide dans le moyen hydraulique.

55. Appareil selon la revendication 54, dans lequel le dispositif de commande (6, 10) comprend une commande à distance sans fil pour commander la soupape.

56. Appareil selon la revendication 53, dans lequel le dispositif de restriction (4, 56) comprend un moyen hydraulique et le dispositif d'actionnement (60) comprend un réservoir (20, 34, 46, 230) formant une chambre de fluide avec un volume variable reliée au moyen hydraulique, et le dispositif d'actionnement (60) est adapté pour distribuer le fluide depuis la chambre vers le moyen hydraulique par réduction du volume de la chambre et pour retirer le fluide du moyen hydraulique vers la chambre par expansion du volume de la chambre.

57. Appareil selon l'une quelconque des revendications 8, 52 et 53, dans lequel le dispositif d'actionnement (60) comprend le moteur (16, 18, 36, 232).

58. Appareil selon la revendication 57, dans lequel le moteur (16, 18, 36, 232) comprend un moteur rotatif, et le dispositif de commande (6, 10) commande le moteur rotatif pour une rotation d'un certain nombre de révolutions.

59. Appareil selon la revendication 57, dans lequel le moteur (16, 18, 36, 232) comprend un moteur linéaire.

60. Appareil selon la revendication 57, dans lequel le moteur (16, 18, 36, 232) comprend un moteur à fluide hydraulique ou pneumatique, et le dispositif de commande (6, 10) commande le moteur à fluide.

61. Appareil selon la revendication 57, dans lequel le moteur (16, 18, 36, 232) comprend un moteur électrique comportant des pièces électriquement conductrices composées de plastique.

62. Appareil selon l'une quelconque des revendications 1, 45 à 53, dans lequel le dispositif de commande (6, 10) libère une énergie polarisée de la source d'énergie.

63. Appareil selon l'une quelconque des revendications 45 à 53, dans lequel le dispositif de commande (6, 10) change la polarité de l'énergie libérée pour inverser le dispositif d'actionnement (60).

64. Appareil selon l'une quelconque des revendications 45 à 53, dans lequel le dispositif d'actionnement (60) comprend un moteur électrique et l'énergie libérée comprend de l'énergie électrique.

65. Appareil selon l'une quelconque des revendications 1, 8, 23, 52, 53 et 57, dans lequel le dispositif de restriction (4, 56) peut être actionné pour réaliser une fonction réversible.

66. Appareil selon la revendication 65, comprenant en outre un dispositif d'inversion (14, 38, 52, 234) pouvant être implanté dans le patient pour inverser la fonction réalisée par le dispositif de restriction (4, 56).

67. Appareil selon la revendication 66, dans lequel le dispositif de commande (6, 10) commande le dispositif d'inversion (14, 38, 52, 234) pour inverser la fonction réalisée par le dispositif de restriction (4, 56).

68. Appareil selon la revendication 66, dans lequel le dispositif d'inversion (14, 38, 52, 234) comprend un moyen hydraulique comprenant une soupape pour changer la direction d'écoulement d'un fluide dans le moyen hydraulique.

69. Appareil selon la revendication 66, dans lequel le dispositif d'inversion comprend un dispositif d'inversion mécanique (14, 52).

70. Appareil selon la revendication 69, dans lequel le dispositif d'inversion mécanique comprend un commutateur (14).

71. Appareil selon la revendication 69, dans lequel le dispositif d'inversion comprend une boîte à engrenage (52).

72. Appareil selon la revendication 66, dans lequel le dispositif d'inversion comprend un commutateur (14).

73. Appareil selon la revendication 72, dans lequel le commutateur du dispositif d'inversion (14) peut être actionné par l'énergie libérée.

74. Appareil selon la revendication 73, dans lequel le dispositif de commande (6, 10) commande l'actionnement du commutateur du dispositif d'inversion (14) en changeant la polarité de l'énergie libérée fournie au commutateur.

75. Appareil selon la revendication 72, dans lequel le commutateur comprend un commutateur électrique et la source d'énergie fournit de l'énergie électrique pour l'actionnement du commutateur.

76. Appareil selon l'une quelconque des revendications 1, 2, 8, 23, 52, 53 et 57, dans lequel le dispositif de restriction (4, 56) comprend un moyen hydraulique et le dispositif d'actionnement (60) est adapté pour conduire un fluide hydraulique dans le moyen hydraulique.

77. Appareil selon la revendication 76, dans lequel le dispositif d'actionnement (60) comprend le moteur (16, 18, 36, 232).

78. Appareil selon la revendication 76 ou 77, dans lequel le dispositif d'actionnement (60) comprend un conduit de fluide relié au moyen hydraulique du dispositif de restriction (4, 56), et un réservoir (20, 34, 46, 230) pour le fluide hydraulique, le réservoir (20, 34, 46, 230) faisant partie du conduit.

79. Appareil selon la revendication 78, dans lequel le moyen hydraulique et le conduit sont dépourvus de toute soupape de non retour.

80. Appareil selon la revendication 79, dans lequel le réservoir (20, 34, 46, 230) forme une chambre de fluide avec un volume variable, et le dispositif d'actionnement (60) est adapté pour distribuer le fluide depuis la chambre vers le moyen hydraulique du dispositif de restriction (4, 56) par réduction du volume de la chambre et pour retirer le fluide du moyen hydraulique vers la chambre par expansion du volume de la chambre.

81. Appareil selon l'une quelconque des revendications précédentes, comprenant en outre au moins un capteur pouvant être implanté (54) pour détecter au moins un paramètre physique du patient.

82. Appareil selon la revendication 81, dans lequel le capteur (54) comprend un capteur de pression pour détecter directement ou indirectement comme paramètre physique la pression dans l'urètre ou la vessie (58).

83. Appareil selon la revendication 81 ou 82, dans lequel le dispositif de commande (6, 10) commande le dispositif de restriction (4, 56) en réponse aux signaux provenant du capteur (54).

84. Appareil selon la revendication 83, dans lequel le dispositif de commande (6, 10) comprend une unité de commande interne (138) pouvant être implantée dans le patient, l'unité de commande interne (138) commandant le dispositif de restriction (4, 56) en réponse aux signaux provenant du capteur (54).

85. Appareil selon la revendication 84, dans lequel le dispositif de commande (6, 10) comprend une unité de commande externe (136) à l'extérieur du corps du patient, l'unité de commande externe (136) commandant le dispositif de restriction (4, 56) en réponse aux signaux provenant du capteur (54).

86. Appareil selon la revendication 85, dans lequel l'unité de commande externe (136) stocke des informations sur le paramètre physique détecté par le capteur (54) et est actionnée manuellement pour commander le dispositif de restriction (4, 56) sur la base des informations stockées.

87. Appareil selon l'une quelconque des revendications 81 à 86, comprenant en outre au moins un émetteur pouvant être implanté pour envoyer des informations sur le paramètre physique détecté par le capteur (54).

88. Appareil selon l'une quelconque des revendications précédentes, dans lequel le dispositif de communication de données externe envoie des données au dispositif de communication de données externe.

89. Appareil selon la revendication 88, dans lequel le dispositif de communication de données interne envoie des données liées au dispositif de restriction (4, 56).

90. Appareil selon la revendication 88 ou 89, dans lequel le dispositif de communication pouvant être implanté envoie des données liées à au moins un signal physique du patient.

91. Appareil selon l'une quelconque des revendications précédentes, dans lequel le dispositif de restriction (4, 56) est adapté pour commander la restriction du passage d'urine.

92. Appareil selon la revendication 91, dans lequel le dispositif de restriction peut être actionné pour élargir et restreindre le passage d'urine lorsqu'il est implanté dans le patient.

93. Appareil selon la revendication 91 ou 92, dans lequel le dispositif de restriction (4, 56) est adapté pour commander progressivement la restriction du passage d'urine lorsqu'il est implanté dans le patient.

94. Appareil selon l'une quelconque des revendications précédentes, comprenant en outre un dispositif de transformation d'énergie pouvant être implanté (6, 62), dans lequel le dispositif de commande (6, 10) est adapté pour commander la source d'énergie pour libérer de l'énergie électrique sans fil, et le dispositif de transformation d'énergie (6, 62) est adapté pour transformer l'énergie électrique en énergie cinétique pour l'actionnement du dispositif de restriction (4, 56).

95. Appareil selon la revendication 94, dans lequel le dispositif de restriction est directement actionné avec l'énergie cinétique, alors que le dispositif de transformation d'énergie (6, 62) transforme l'énergie électrique en énergie cinétique.

96. Appareil selon l'une quelconque des revendications précédentes, dans lequel le dispositif de commande (6, 10) commande la source d'énergie pour libérer l'énergie magnétique, l'énergie non magnétique, l'énergie électromagnétique, l'énergie non électromagnétique, l'énergie cinétique, l'énergie non cinétique, l'énergie sonique, l'énergie non sonique, l'énergie thermique ou l'énergie non thermique.

97. Appareil selon l'une quelconque des revendications précédentes, dans lequel le dispositif de restriction (4, 56) est non gonflable.

98. Appareil selon l'une quelconque des revendications précédentes, dans lequel le dispositif de commande (6, 10) commande la source d'énergie pour libérer de l'énergie pour une période prédéterminée.

99. Appareil selon l'une quelconque des revendications 1 à 97, dans lequel le dispositif de commande (6, 10) commande la source d'énergie pour libérer de l'énergie selon un nombre d'impulsions d'énergie déterminé.

100. Appareil selon l'une quelconque des revendications précédentes, dans lequel le dispositif de commande (6, 10) est adapté pour commander la source d'énergie pour libérer de l'énergie de manière non invasive.

101. Appareil selon l'une quelconque des revendications précédentes, dans lequel le dispositif de commande (6, 10) comprend une commande à distance sans fil pour transmettre au moins un signal de commande sans fil pour commander le dispositif de restriction (4, 56).

102. Appareil selon la revendication 101, dans lequel la commande à distance est capable d'obtenir des informations sur la condition du dispositif de restriction (4, 56) lorsque le dispositif de restriction (4, 56) peut être implanté et pour commander le dispositif de restriction (4, 56) en réponse aux informations.

103. Appareil selon la revendication 101 ou 102, dans lequel la commande à distance sans fil comprend au moins un transmetteur ou un transducteur de signal externe ou et au moins un transmetteur ou un transducteur de signal interne pouvant être implanté dans le patient.

104. Appareil selon la revendication 101 ou 102, dans lequel la commande à distance sans fil comprend au moins un transmetteur ou un transducteur de signal externe ou et au moins un transmetteur ou un transducteur de signal interne pouvant être implanté dans le patient.

105. Appareil selon l'une quelconque des revendications 101 à 104, dans lequel la commande à distance est capable d'envoyer des informations liées au dispositif de restriction (4, 56) depuis l'intérieur du corps du patient vers l'extérieur de celui-ci.

106. Appareil selon la revendication 105, dans lequel la commande à distance commande le dispositif de restriction (4, 56) en réponse aux informations.

107. Appareil selon l'une quelconque des revendications 102 à 106, dans lequel la commande à distance transmet un signal porteur pour transporter le signal de commande.

108. Appareil selon la revendication 107, dans lequel le signal porteur peut être modulé en fonction de la fréquence, de l'amplitude ou de la fréquence et de l'amplitude.

109. Appareil selon la revendication 107 ou 108, dans lequel le signal porteur est numérique, analogique ou numérique et analogique.

110. Appareil selon l'une quelconque des revendications 107 à 109, dans lequel le signal de commande utilisé avec le signal porteur est modulé en fonction de la fréquence, de l'amplitude ou de la fréquence et de l'amplitude.

111. Appareil selon l'une quelconque des revendications 101 à 110, dans lequel le signal de commande comprend un signal d'onde comprenant un signal parmi un signal d'onde sonore incluant un signal d'onde ultrasonore, un signal d'onde électromagnétique comprenant un signal lumineux infrarouge, un signal lumineux visible, un signal lumineux ultraviolet et un signal lumineux laser, un signal de micro-onde, un signal d'onde radio, un signal de rayonnement à rayon x, et un signal de rayonnement gamma.

112. Appareil selon l'une quelconque des revendications 101 à 110, dans lequel le signal de commande comprend un champ électrique, magnétique ou électrique et magnétique.

113. Appareil selon l'une quelconque des revendications 101 à 111, dans lequel le signal de commande est numérique, analogique ou numérique et analogique.

114. Appareil selon la revendication 113, dans lequel la commande à distance transmet un signal d'onde porteur électromagnétique pour transporter le signal de commande numérique ou analogique.

115. Appareil selon l'une quelconque des revendications 101 à 114, dans lequel le signal de commande est transmis en impulsions par la commande à distance sans fil.

116. Appareil selon la revendication 2 ou 3, comprenant en outre un stabilisateur pouvant être implanté pour stabiliser l'énergie libérée par le dispositif de commande (6, 10).

117. Appareil selon la revendication 116, dans lequel l'énergie libérée par le dispositif de commande (6, 10) comprend de l'énergie électrique et le stabilisateur comprend au moins un condensateur (162).

118. Appareil selon la revendication 1, dans lequel le dispositif de commande (6, 10) est adapté pour commander la source d'énergie pour libérer de l'énergie pour une utilisation directe en liaison avec l'actionnement du dispositif de restriction (4, 56).

119. Appareil selon la revendication 1, dans lequel le dispositif de commande (6, 10) est adapté pour commander la source d'énergie pour libérer de l'énergie par intermittence sous la forme d'un train d'impulsions d'énergie pour une utilisation directe en liaison avec l'actionnement du dispositif de restriction (4, 56).

120. Appareil selon la revendication 4 ou 119, dans lequel le dispositif de commande (6, 10) est adapté pour commander la source d'énergie pour libérer de l'énergie électrique, et comprenant en outre un condensateur pouvant être implanté pour produire le train d'impulsions d'énergie à partir de l'énergie libérée.

121. Appareil selon la revendication 1, comprenant en outre des composants électriques pouvant être implantés comprenant au moins un dispositif de contrôle de la tension.

122. Appareil selon la revendication 1, comprenant en outre des composants électriques pouvant être implantés comprenant un unique dispositif de contrôle de la tension unique.

123. Appareil selon la revendication 121 ou 122, dans lequel les composants électriques sont dépourvus de tout détecteur de courant et/ou de détecteur de niveau de charge.

124. Appareil selon l'une quelconque des revendications 120 à 123, comprenant en outre un condensateur ou un accumulateur pouvant être implanté, dans lequel la charge ou la décharge du condensateur ou de l'accumulateur est commandée par l'utilisation du dispositif de contrôle de la tension.

125. Appareil selon la revendication 35, dans lequel le dispositif de commande (6, 10) commande la source d'énergie externe pour libérer de l'énergie sans fil.

126. Appareil selon la revendication 116, dans lequel l'énergie libérée comprend de l'énergie électrique et comprenant en outre un condensateur pouvant être implanté pour produire le train d'impulsions d'énergie.

127. Appareil selon la revendication 116 ou 124, dans lequel le condensateur a une capacité inférieure à 0,1 µF.

128. Appareil selon l'une quelconque des revendications 1 à 3 et 116, dans lequel le dispositif de commande (6, 10) est adapté pour commander la source d'énergie pour alimenter directement le moteur (16, 18, 36, 232) avec l'énergie libérée.

129. Appareil selon la revendication 23, dans lequel l'énergie sans fil comprend des ondes électromagnétiques en excluant les ondes radio.

130. Appareil selon l'une quelconque des revendications 2, 3 et 118, dans lequel le dispositif de commande (6, 10) est adapté pour libérer de l'énergie sans fil sous la forme d'un champ magnétique ou d'ondes électromagnétiques pour une alimentation directe du moteur (16, 18, 36, 232), alors que l'énergie sans fil est libérée.

131. Appareil selon l'une quelconque des revendications 2 à 4, dans lequel l'énergie sans fil comprend un signal.

132. Appareil selon la revendication 1, comprenant en outre un dispositif de transformation d'énergie pouvant être implanté (6, 62) pour transformer de l'énergie sans fil directement ou indirectement en une énergie différente de l'énergie sans fil pour l'actionnement du dispositif de restriction (4, 56).

133. Appareil selon la revendication 132, dans lequel le dispositif de transformation d'énergie (6, 62) transforme de l'énergie sans fil sous la forme d'ondes sonores en énergie électrique pour l'actionnement du dispositif de restriction.

134. Appareil selon la revendication 133, dans lequel le dispositif de transformation d'énergie (6, 62) transforme de l'énergie sans fil sous la forme d'ondes sonores directement en énergie électrique.

135. Appareil selon l'une quelconque des revendications 133 ou 134, dans lequel le dispositif de transformation d'énergie (6, 62) comprend un condensateur.

136. Appareil selon la revendication 135, dans lequel le condensateur est adapté pour produire des impulsions électriques à partir de l'énergie électrique transformée.

137. Appareil selon l'une quelconque des revendications 132 à 136, dans lequel le moteur (16, 18, 36, 232) est alimenté par l'énergie transformée.

138. Appareil selon la revendication 1, dans lequel le dispositif de restriction (4, 56) est réglable de manière non manuelle.

139. Appareil selon l'une quelconque des revendications précédentes, dans lequel le dispositif de restriction (4, 56) est incorporé dans un matériau mou ou semblable à du gel.

140. Appareil selon l'une quelconque des revendications précédentes, dans lequel le dispositif de restriction (4, 56) est incorporé dans un matériau siliconé ayant une dureté inférieure à 20 Shore.

141. Appareil selon la revendication 3, comprenant en outre une source d'énergie pouvant être activée pouvant être implantée dans le patient, dans lequel la source d'énergie pouvant être implantée est activée par l'énergie sans fil libérée de la source d'énergie externe, pour fournir de l'énergie qui est utilisée en liaison avec l'actionnement du dispositif de restriction (4, 56).

142. Appareil selon l'une quelconque des revendications précédentes, dans lequel le dispositif de commande (6, 10) est adapté pour commander le moteur ou la pompe (16, 18, 36, 232) en libérant de l'énergie pour actionner le moteur ou la pompe pour actionner le dispositif de restriction (4, 56) pour à la fois restreindre le passage d'urine et inverser la fonction réalisée par le dispositif de restriction (4, 56).
